# EUROPEAN PATENT APPLICATION

(11) **EP 3 949 974 A1**
(43) Date of publication of application: **09.02.2022**
(21) Application number: 20777651.9
(22) Date of filing: 27.03.2020
(51) Int. Cl.: A61K 35/64, A61K 38/46, A61P 25/28, A23L 33/10

(54) **COMPOSITION FOR PREVENTING OR TREATING NEUROINFLAMMATORY DISORDERS, COMPRISING BEE VENOM EXTRACT AS ACTIVE INGREDIENT**

(30) Priority: 28.03.2019 KR 20190036092; 26.03.2020 KR 20200036912
(71) Applicant: Inist St Co., Ltd., Chungcheongbuk-do 27644 (KR)
(72) Inventor: KIM, Kuk Hyun, Yongin-si Gyeonggi-do 16824 (KR); RHEE, Hae In, Yongin-si Gyeonggi-do 16936 (KR)
(74) Representative: Roos, Peter
(86) International application number: PCT/KR2020/004239
(87) International publication number: WO 2020/197332

(57) **Abstract**

The present invention relates to a composition for preventing or treating neuroinflammatory disorders, comprising a bee venom extract as an active ingredient. More specifically, the present invention provides a pharmaceutical composition or a health functional food composition for preventing or treating neuroinflammatory disorders, both of which comprise, as an active ingredient, a bee venom extract in which the amount of phospholipase A2 (PLA2) is 50 to 85%. The bee venom extract alleviates decreased motor functions caused by neurotoxicity and has neuroprotective and neuroinflammation-relieving effects, thereby enabling the prevention or treatment of neuroinflammatory disorders. Particularly, a pharmaceutical composition according to the present invention can further improve an effect of preventing or treating neuroinflammatory disorders by being subcutaneously administered or by being co-administered with a STAT3 inhibitor or an NF-κB inhibitor, thereby enabling more effective prevention or treatment of neuroinflammatory disorders.

## Description

### TECHNICAL FIELD

The present invention relates to a composition for preventing or treating neuroinflammatory disorders, comprising a bee venom extract as an active ingredient.

### BACKGROUND ART

Chronic inflammation in the central nervous system (CNS) is closely related to a variety of pathophysiological processes, including metabolic complications and neurodegenerative diseases. Activated microglia is detected in areas such as hypothalamus and hippocampus under chronic inflammatory conditions, and activated microglia-derived inflammatory mediators cause nerve damage, affecting metabolic disorders and neurodegenerative diseases. Accordingly, inhibition of microglia activity is considered as an important strategy for protecting an aberrant chronic inflammatory response in the central nervous system, such as the hypothalamus and/or hippocampus.

Alzheimer's disease (AD) is the most common neurodegenerative disease that causes dementia, its main symptoms include memory declines, language deficits, and confusions about time and place. Although etiology and mechanism of Alzheimer's disease have not yet been precisely elucidated, senile plaques and neurofibrillary tangles have been observed in the patients' brain, and it has been found that the neurofibrillary tangles are formed by neuronal cell death, synaptic loss, and denaturation and hyperphosphorylation of tau protein by toxic proteins such as beta-amyloid (Aβ) and APP-C protein (anti-amyloid precursor protein).

Beta-amyloid (Aβ), a major component of amyloid plaques found in the brain of Alzheimer's disease patients, is a peptide consisting of 36 to 43 amino acids and is known to be made from amyloid precursor protein (APP). Amyloid precursor protein is degraded by β- and γ-secretase, and studies have been conducted to reduce the concentration of beta-amyloid by targeting the degradative enzyme. However, therapeutic substances targeting the beta-amyloid production mechanism have insignificant therapeutic effects on Alzheimer's disease.

In addition, after active and passive immunotherapy showed therapeutic effects in animal experiments, in a clinical study on Alzheimer's patients, in the case that active immunity was induced by the antigen Aβ 42 that used in an animal model of Alzheimer's disease, Aβ plaques were reduced and the Morris water maze experiment showed improvement in memory. However, in a phase 2 clinical trial for Alzheimer's patients using the synthetic Aβ peptide AN1792/QS-21, 6% of the patients showed meningitis, so the clinical trial was discontinued. A recent clinical trial also reduced the Aβ plaques, but did not improve progressive neuronal degeneration.

Parkinson's disease (PD) is one of the representative neurodegenerative disorders that appear in old age along with Alzheimer's disease, and it has been known that 1% of the population over the age of 65 gets the disease and the rate of the disease increases with advancing age. Parkinson's disease is a movement disorder, and typical symptoms include tremor at rest, rigidity, bradykinesia, and postural instability. In addition, Parkinson's disease is characterized by microgliosis, astrogliosis, progressive degeneration of dopaminergic neurons, the presence of Lewy bodies in dopaminergic neurons, and accumulation of α-synuclein in substantia nigra pars compacta.

Although the exact cause of Parkinson's disease is not yet known clearly, it is understood that environmental factors caused by neurotoxins such as pesticides, genetic factors, mitochondrial dysfunction, and aging are related to it. In particular, activated microglia and neuroinflammation are reported to play an important role in the pathogenesis of these neurodegenerative disorders.

Currently, although there are a number of drugs that relieve symptoms of Parkinson's disease, such as L-dopa preparations, dopamine receptor agonists, anticholinergic drugs, and Eldepryl, these drugs only play a role in controlling the symptoms rather than radical therapy. No drugs which can stop the progression of the disease have yet been reported.

In addition, since these drugs require continuous administration, chronic use of these drugs has a high risk of causing side effects that weaken the mind and body. For example, anticholinergic drugs may cause abnormalities in the autonomic nervous system or mental function, so there is a limit to continuous administration to elderly patients. In the case of L-dopa preparations, the effect gradually decreases with long-term use, and side effects such as twisting of the body or abnormal movement of hands and feet occur. In addition, a nerve stimulation therapy using high frequency, that is, a surgical treatment method such as high frequency destruction or deep brain stimulation therapy is also performed, but there are some problems, which invasive surgery is required and a lot of cost is consumed.

Therefore, for the prevention or treatment of disorders such as Alzheimer's disease, Parkinson's disease, and stroke, there is an urgent need to develop more effective new therapeutic agents.

### DISCLOSURE

### TECHNICAL PROBLEM

It is an object of the present invention to provide a pharmaceutical composition for fundamentally preventing or treating neuroinflammatory disorders.

Another object of the present invention is to provide a method for subcutaneous administration of the composition for preventing or treating neuroinflammatory disorders.

Another object of the present invention is to provide a functional food composition for preventing or improving neuroinflammatory disorders.

### TECHNICAL SOLUTION

In order to achieve the above objects, a pharmaceutical composition for preventing or treating neuroinflammatory disorders according to the present invention may comprise a bee venom extract having a phospholipase A2 (PLA2) content of 50 to 85% as an active ingredient.

A pharmaceutical composition for subcutaneous administration for preventing or treating neuroinflammatory disorders according to the present invention may comprise a bee venom extract having a phospholipase A2 (PLA2) content of 50 to 85% as an active ingredient.

A pharmaceutical composition for preventing or treating neuroinflammatory disorders according to the present invention may include a pharmaceutical composition comprising a bee venom extract having a PLA2 content of 50 to 85% as an active ingredient, and one or more inhibitors selected from STAT3 inhibitors and NF-κB inhibitors.

A pharmaceutical composition according to the present invention may be administered subcutaneously for preventing or treating neuroinflammatory disorders.

A functional food composition for preventing or treating neuroinflammatory disorders according to the present invention may comprise a bee venom extract having a phospholipase A2 (PLA2) content of 50 to 85% as an active ingredient.

In addition, a functional food composition for preventing or improving neuroinflammatory disorders according to the present invention is a functional food composition comprising a bee venom extract having the PLA2 content of 50 to 85% as an active ingredient, and one or more inhibitors selected from STAT3 inhibitors and NF-κB inhibitors.

### ADVANTAGEOUS EFFECTS

The pharmaceutical composition or the functional food composition according to the present invention comprises a bee venom extract having a phospholipase A2 (PLA2) content of 50 to 85% as an active ingredient, thereby improving motor function deterioration due to neurotoxicity, and having a neuroprotective effect through reduction in polarization of Th1 and Th17 and increase in TH-positive dopaminergic neurons. Further, it results in a better neuroinflammatory alleviation effect by reducing the expression of pro-inflammatory cytokines and regulating the neuroinflammatory response by activating regulatory T cells.

In addition, the pharmaceutical composition according to the present invention can be administered subcutaneously or in combination with a STAT3 inhibitor or an NF-κB inhibitor to further enhance the prevention or treatment effect of a neuroinflammatory disease, thereby preventing or treating the neuroinflammatory disease more effectively.

### BRIEF DESCRIPTIONS OF DRAWINGS

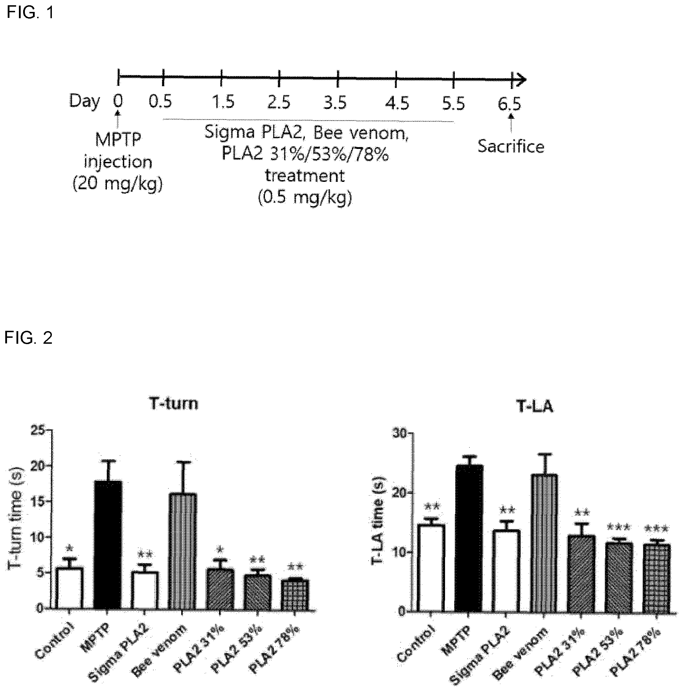

FIG. 1 shows an animal experiment schedule with subcutaneous injection of 0.5mg/kg of each of Sigma PLA2, crude Bee Venom, a bee venom extract containing PLA2 31% (PLA2 31%), a bee venom extract containing PLA2 53% (PLA2 53%), and a bee venom extract containing PLA2 78% (PLA2 78%) for 6 days after intraperitoneal injection of MPTP (1-Methyl-4-phenyl-1,2,3,6-tetrahydropyridinehydrochloride, 20 mg/kg) in mice for evaluation of efficacy according to PLA2 (Phospholipase A2) content.
FIG. 2 shows graphs measuring the behavioral experiment by a pole test, indicating effects of the PLA2 content of bee venom extracts for the values of the time taken for the MPTP-injected mice to turn completely downward (T-turn, left) and the total time taken for the mice to reach the floor (T-LA, right). Error bars represent mean±SEM, *P < 0.05, **P < 0.01, and ***P < 0.001, indicating significant differences with subtantia nigra (SN) of the MPTP-injected mice.

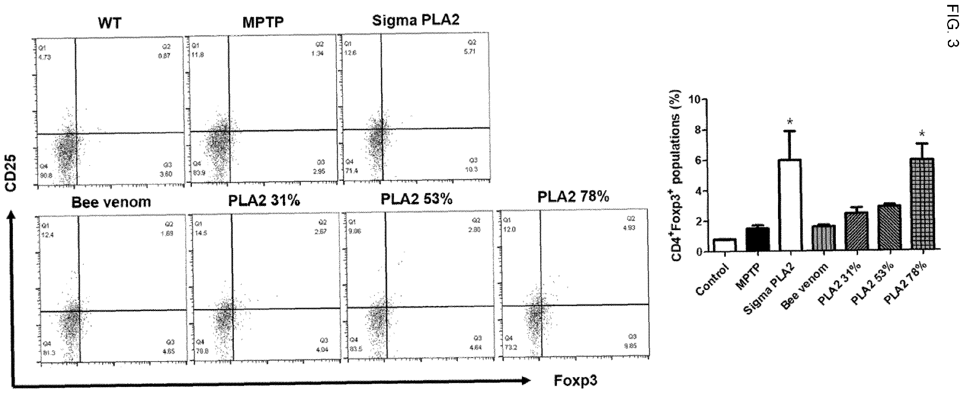

FIG. 3 shows effects of the PLA2 content of bee venom extracts on differentiation of regulatory T cells (Tregs) in the mice injected with MPTP. Treg differentiation in spleen cells was measured by flow cytometry for cells expressing CD4, CD25, and Foxp3. Error bars represent mean±SEM and indicate significant differences from the MPTP-injected mice by *P < 0.05, and the significance was determined by Student's t-test.

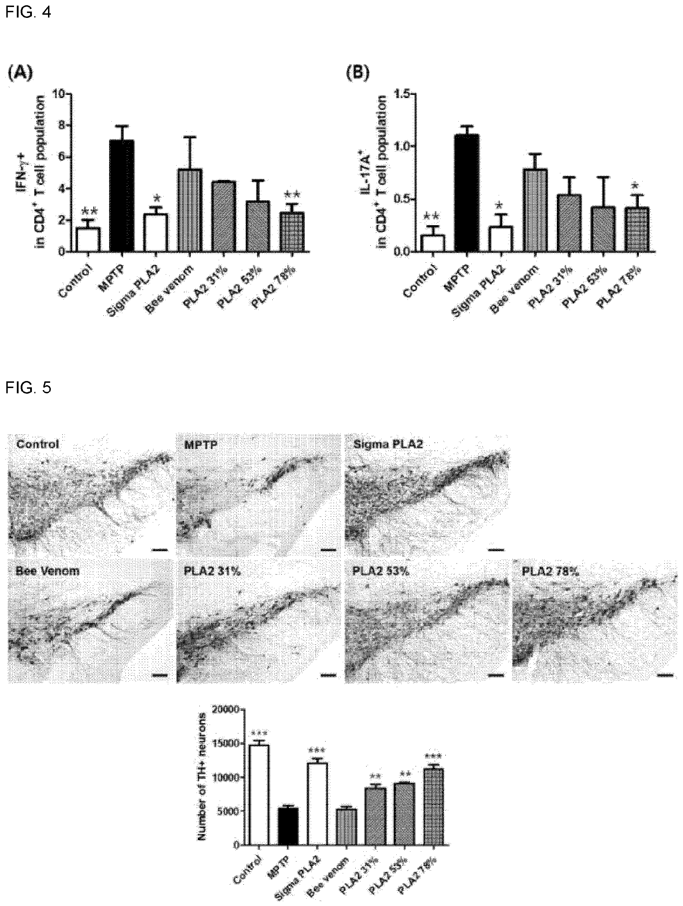

FIG. 4 shows effects of the PLA2 content of bee venom extracts on differentiation of Th1/17 cells in the MPTP-injected mice. Th1/17 cell differentiation in spleen cells was measured by flow cytometry for (A) cells expressing Th1 (CD4+IFN-y+) and (B) cells expressing Th17 (CD4+IL-17A+). Error bars represent mean±SEM and indicate significant differences from the MPTP-injected mice by *P < 0.05, **P < 0.01.
FIG. 5 shows immunohistochemical staining to identify tyrosine hydroxylase (TH) from a substantia nigra (SN) tissue sample of a group treated with bee venom extracts having different PLA2 content. Brain tissues were stained with TH for damage analysis on dopaminergic nerve cells, and the number of TH-positive neurons in the SN was quantified using a semi-quantitative scale. All randomly selected histological images were scored, the data was representative of three independent experiments and presented as mean±SEM, and scale bars are 5.0 µm. **P < 0.01, ***P < 0.001 indicate significant differences from the MPTP group.

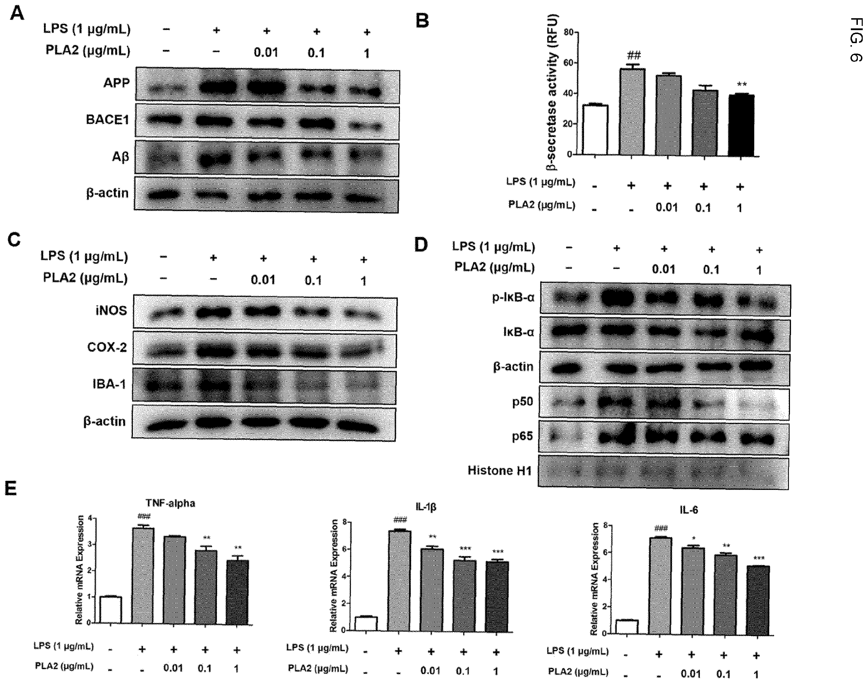

FIG. 6 shows effects of the bee venom extract on LPS-induced amyloid production and neuroinflammation in microglial cells. FIG. 6A shows Western blot analysis results on amyloid precursor protein (APP), beta-secretase 1 (BACE1), and β-Amyloid (Aβ) with bvPLA2 (PLA2) treatment. FIG. 6B depicts a graph of β-secretase activity analysis results with bvPLA2 treatment. FIG. 6C shows Western blot analysis results of iNOS, COX-2, and IBA-1 with bvPLA2 treatment. FIG. 6D shows Western blot analysis results of NF-κB subunits. FIG. 6E shows qRT-PCR analysis results of proinflammatory cytokines with bvPLA2 treatment.

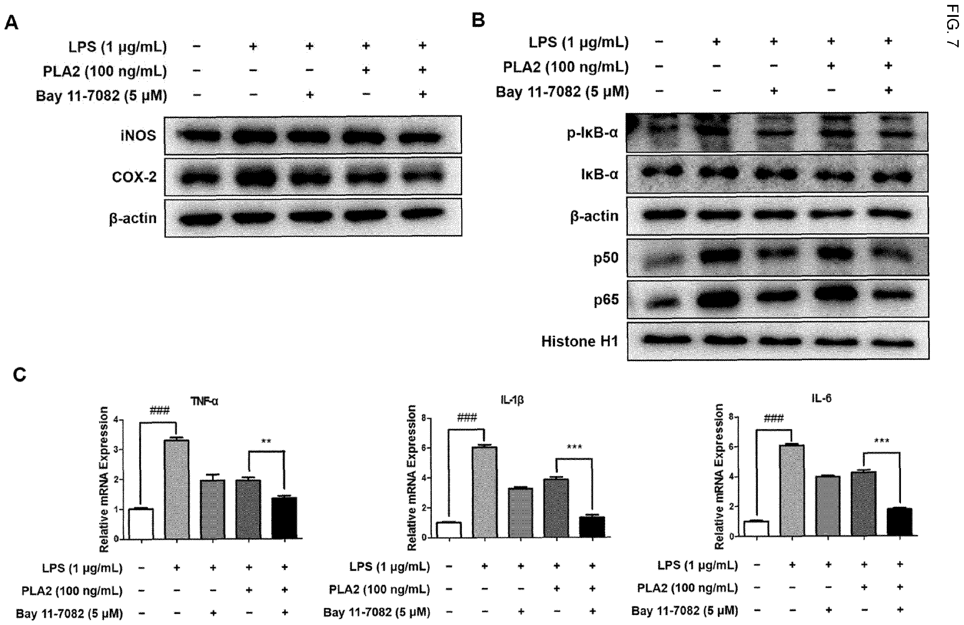

FIG. 7 shows identification of combination effects of the bee venom extract and an NF-κB inhibitor (Bay 11-7802). FIG. 7A shows the effects of iNOS and COX-2 on inflammatory proteins, FIG. 7B shows the effects on NF-κB signaling pathway related factors, and FIG. 7C shows the effects on pro-inflammatory cytokines.

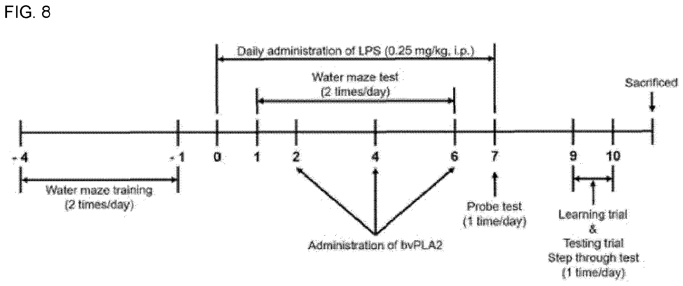

FIG. 8 shows a schematic procedure of an in vivo experiment.

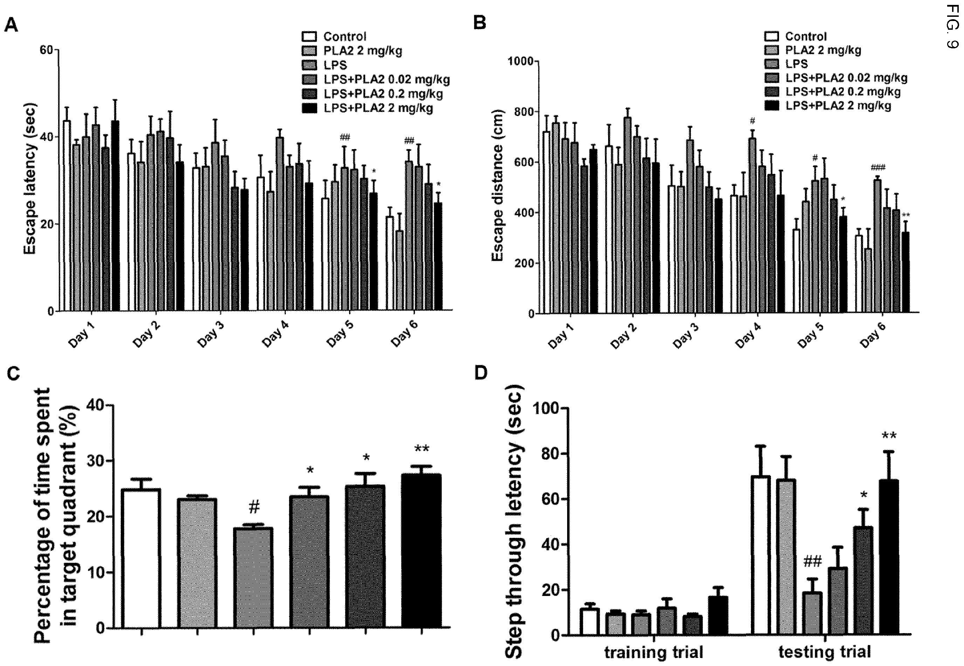

FIG. 9 shows effects of the bee venom extract on improvement of memory in LPS-induced mice. FIGS. 9A and 9B show graphs of results of a Morris water maze experiment, FIG. 9C shows a graph of a probe test result, and FIG. 9D shows a graph of a result of a passive avoidance experiment.

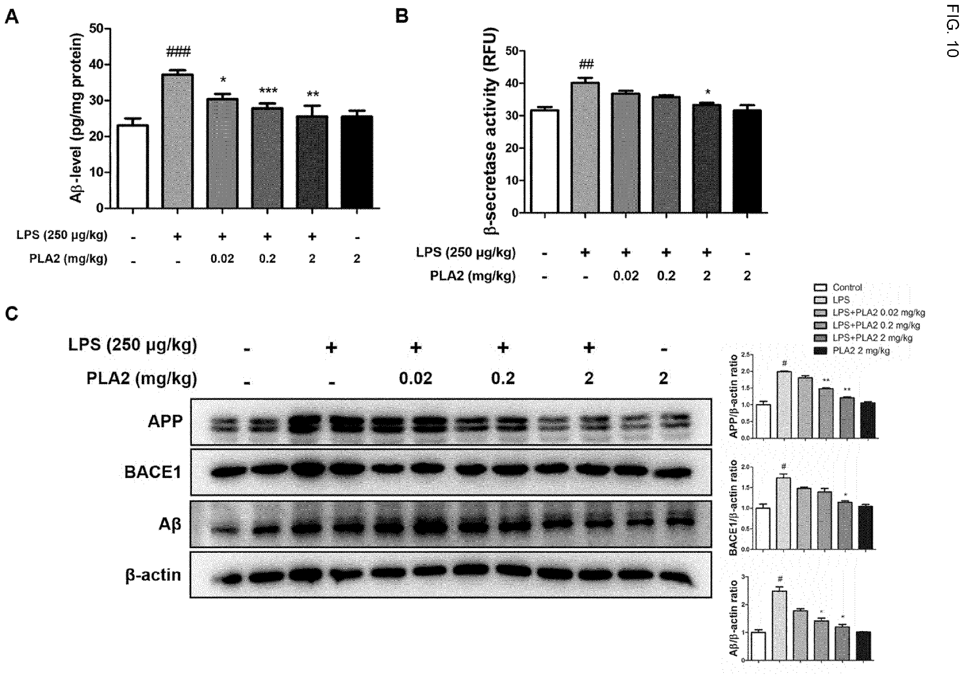

FIG. 10 shows effects of the bee venom extract on reduction of amyloidosis in brain tissues of LPS-induced mice. FIG. 10A shows a graph of changes in β-amyloid production with bvPLA2 treatment, Fig. 10B shows a graph of analysis results on β-secretase activity with the bvPLA2 treatment, and FIG. 10C shows Western blot analysis results of APP, BACE1, and β-amyloid (Aβ) with the bvPLA2 treatment.

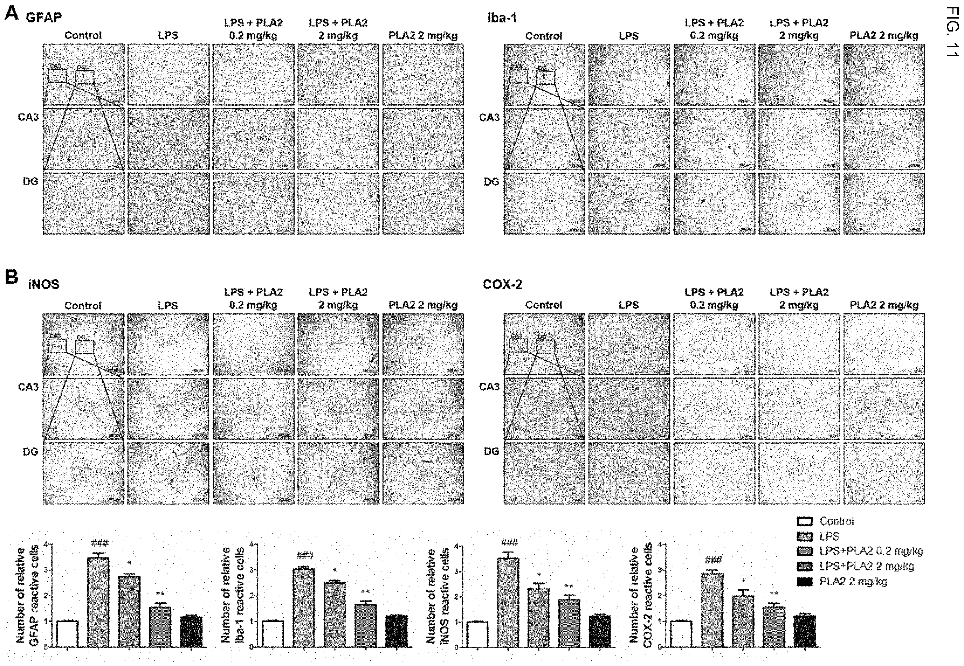

FIG. 11 shows effects of the bee venom extract on neuroinflammation in the brain of LPS-induced mice. FIG. 11A shows changes in the number of reactive cells of GFAP and IBA-1, and FIG. 11B shows changes in the number of reactive cells of iNOS and COX-2.

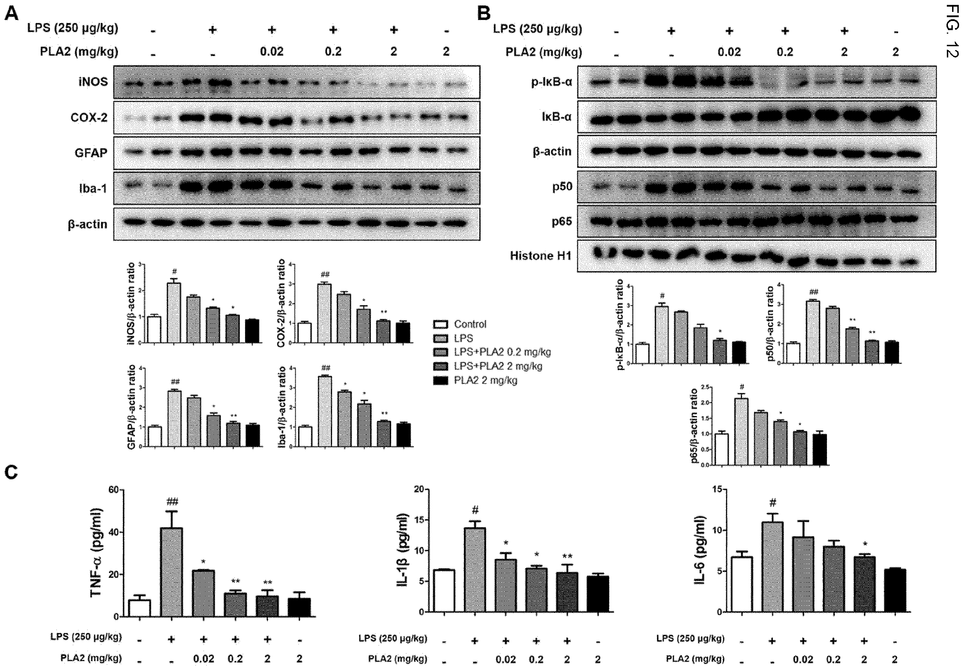

FIG. 12 shows effects of the bee venom extract on inflammatory response and pro-inflammatory cytokines in the brain tissues of LPS-induced mice. FIG. 12A shows Western blot analysis results of iNOS, COX-2, GFAP, and IBA-1 with bvPLA2 treatment, FIG. 12B shows Western blot analysis results of NF-κB subunits and the like with the bvPLA2 treatment, and FIG. 12C shows graphs indicating changes in the expression level of pro-inflammatory cytokines with the bvPLA2 treatment.

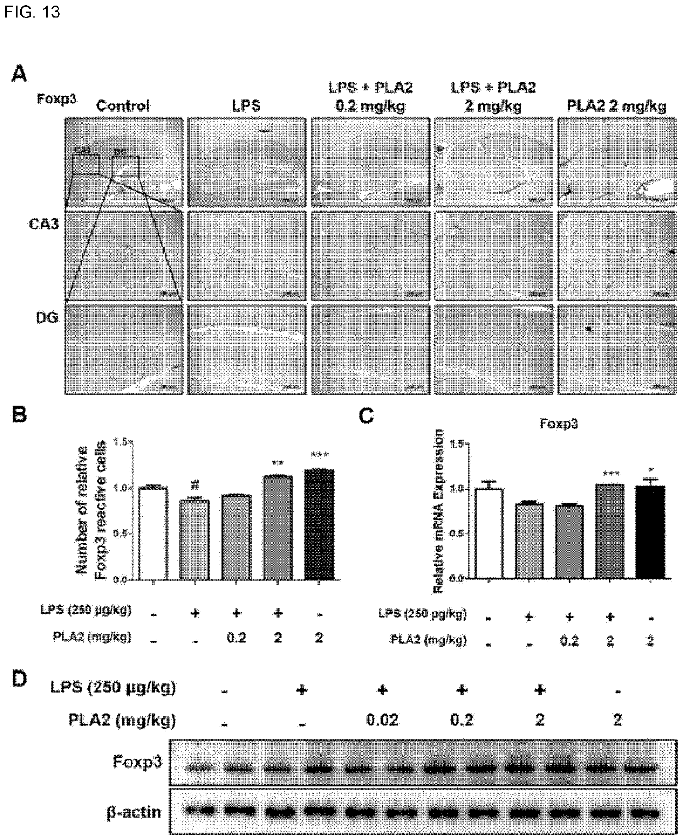

FIG. 13 shows effects of the bee venom extract on regulatory T cells (Treg) in brain tissues of LPS-induced mice. FIGS. 13A and 13B show immunohistochemical results of Foxp3 which is a Treg marker, and FIG. 13C shows expression levels of Foxp3 with qRT-PCR.

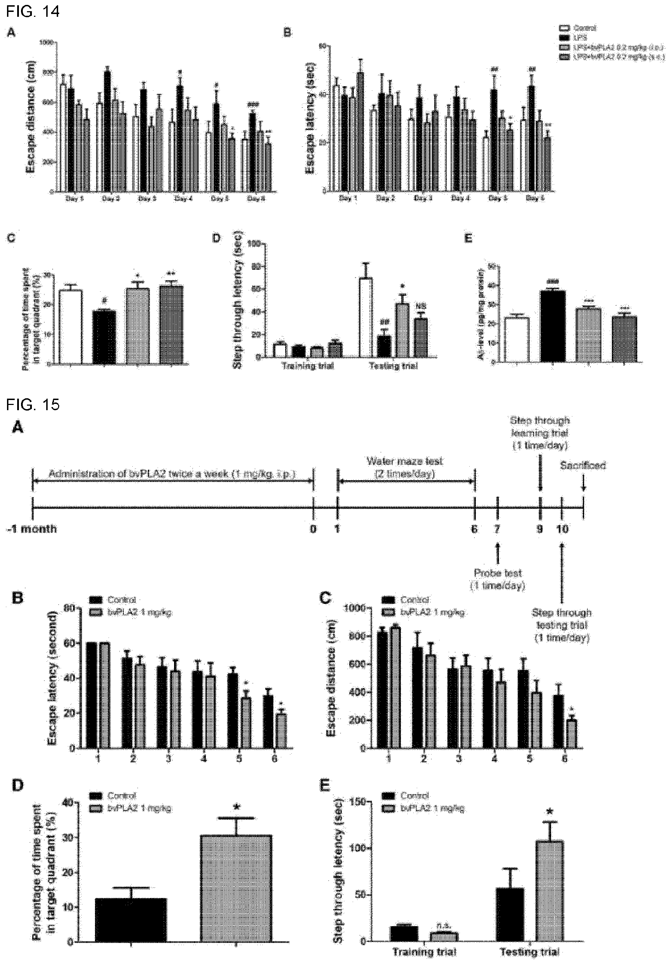

FIG. 14 shows effects on improvement of memory of LPS-induced mice according to routes of administration of the bee venom extract. FIGS. 14A to 14D show results of behavioral experiments according to the routes of administration, and FIG. 14E shows comparison of changes in amyloidosis according to the routes of administration.
FIG. 15 shows effects of the bee venom extract on genetically induced memory impairment in Tg2576 mice. FIG. 15A schematically shows an experimental procedure with bvPLA2 administration, and FIGS. 15B to 15D show behavioral experimental results with bvPLA2 treatment. Each value represents the mean±SEM of 10 mice, *p < 0.05 indicating significant differences from a control group.

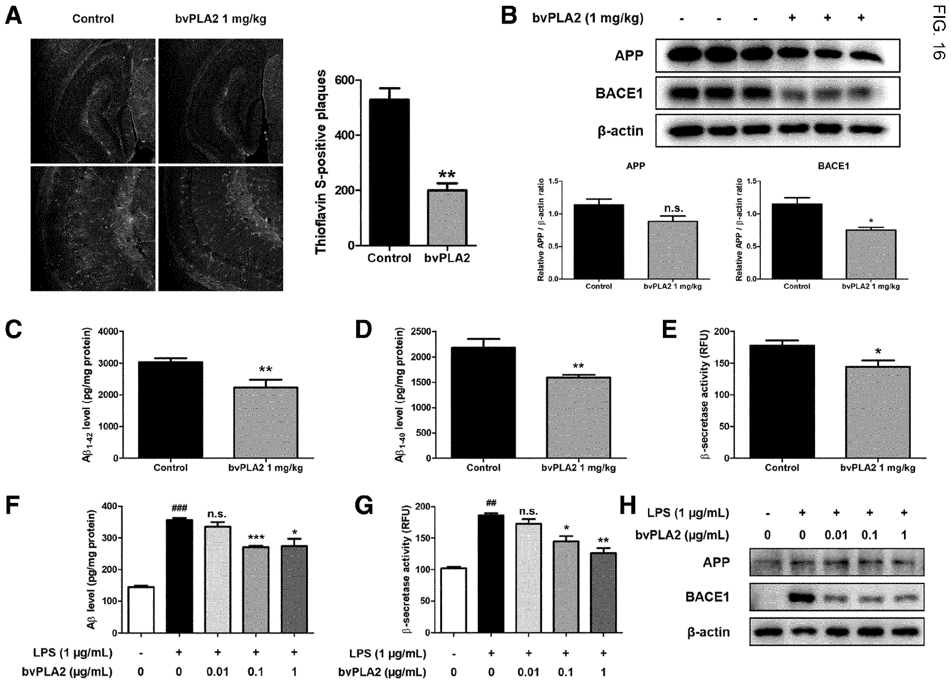

FIG. 16 shows inhibitory effects of the bee venom extract on β-amyloid (Aβ) accumulation and factors related to Aβ production. FIG. 16A shows results of Thioflavin S staining, FIG. 16B shows Western blot analysis results of APP and BACE1, FIGS. 16C and 16D show results of evaluating levels of Ap₁₋₄₂ and Aβ₁₋₄₀ by ELISA, and FIG. 16E shows analysis results of β-secretase activity. Each value represents the mean±SEM of 10 mice, and **p < 0.01 indicates significant differences from a control group. FIG. 16F shows results of evaluating an Ap₁₋₄₂ level in BV-2 microglia by ELISA, and FIG. 16G shows analysis results of β-secretase activity in BV-2 cells, and each value indicates the mean±SEM of 3 mice. FIG. 16H shows Western blot results of APP and BACE1 in BV-2 cells, ##p < 0.05, ###p < 0.001 indicate significant differences from a control group, and *p < 0.05, **p < 0.01, ***p < 0.001 indicate significant differences from the LPS group.

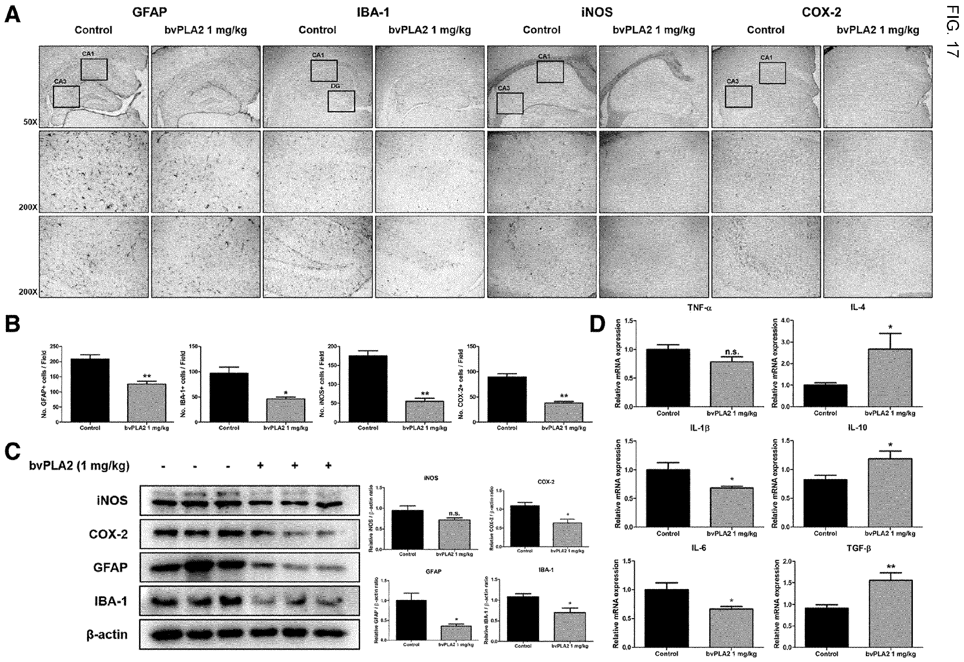

FIG. 17 shows inhibitory effects of the bee venom extract on genetically induced neuroinflammation in Tg2576 mice. FIG. 17A shows immunostaining results of GFAP, IBA-1, iNOS, and COX-2 in the hippocampus, FIG. 17B shows quantification of the number of positive cells from the immunostaining, FIG. 17C shows results of Western blotting of iNOS, COX-2, GFAP, and IBA-1, and FIG. 17D show results of evaluating their expression levels by qRT-PCR. Each value represents the mean±SEM of 10 mice, *p < 0.05, **p < 0.01, indicating significant differences from a control group.

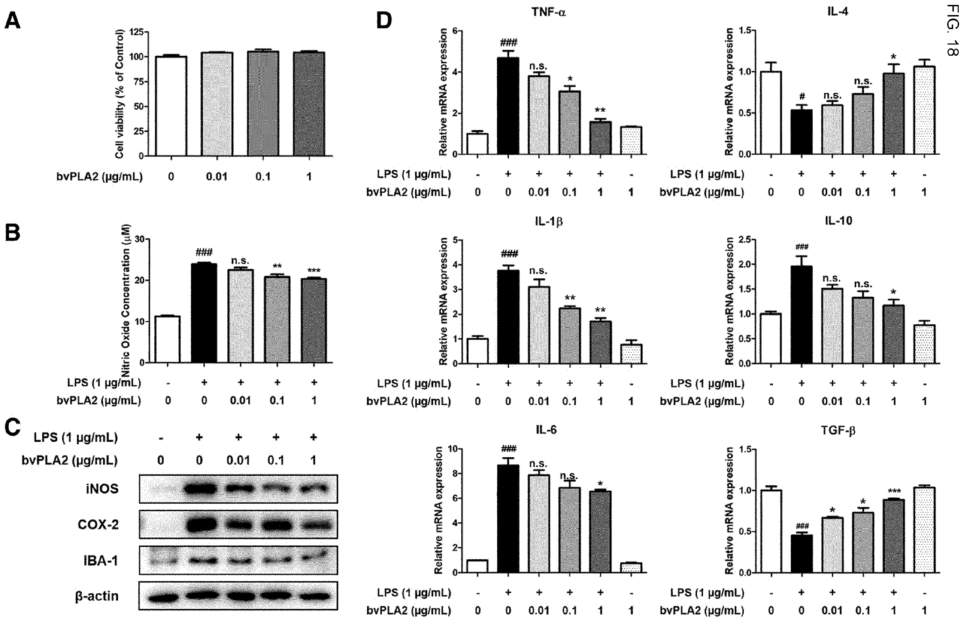

FIG. 18 shows inhibitory effects of the bee venom extract on LPS-induced neuroinflammation in BV-2 cells. FIG. 18A shows cell viability analysis results, FIG. 18B shows nitric oxide assay, FIG. 18C shows iNOS, COX-2, and IBA -1 Western blot results, and FIG. 18D shows results of evaluating pro-inflammatory cytokines and anti-inflammatory cytokines by qRT-PCR. Each value represents the mean±SEM of three different experiments. #p < 0.05, ###p < 0.001 indicate significant differences from a control group, and *p < 0.05, **p < 0.01, ***p < 0.001 indicate significant differences from the LPS group.

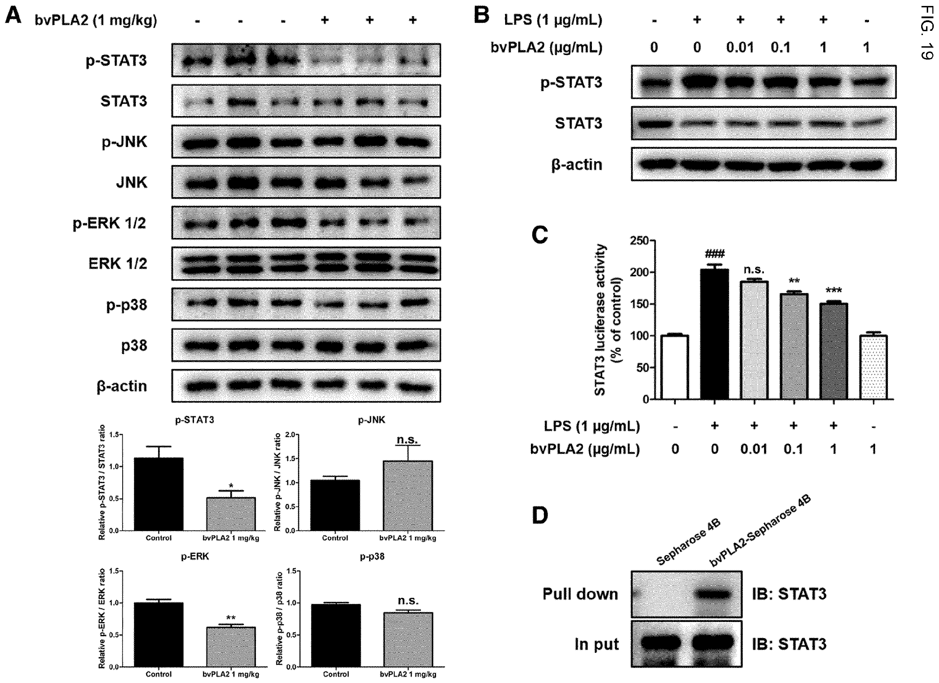

FIG. 19 shows inhibitory effects of the bee venom extract on STAT3 activation. FIG. 19A shows Western blotting results on the levels of factors related to STAT3 and mitogen-activated protein kinases (MAPK) signaling pathways in the brain of Tg2576 mice, *p < 0.05, **p < 0.01, indicating significant differences from a control group. FIG. 19B shows Western blotting results in BV-2 cells, and FIG. 19C shows luciferase activity assay results, each value representing the mean±SEM of three different experiments. FIG. 19D shows results of Western blot analysis of the level of STAT3.

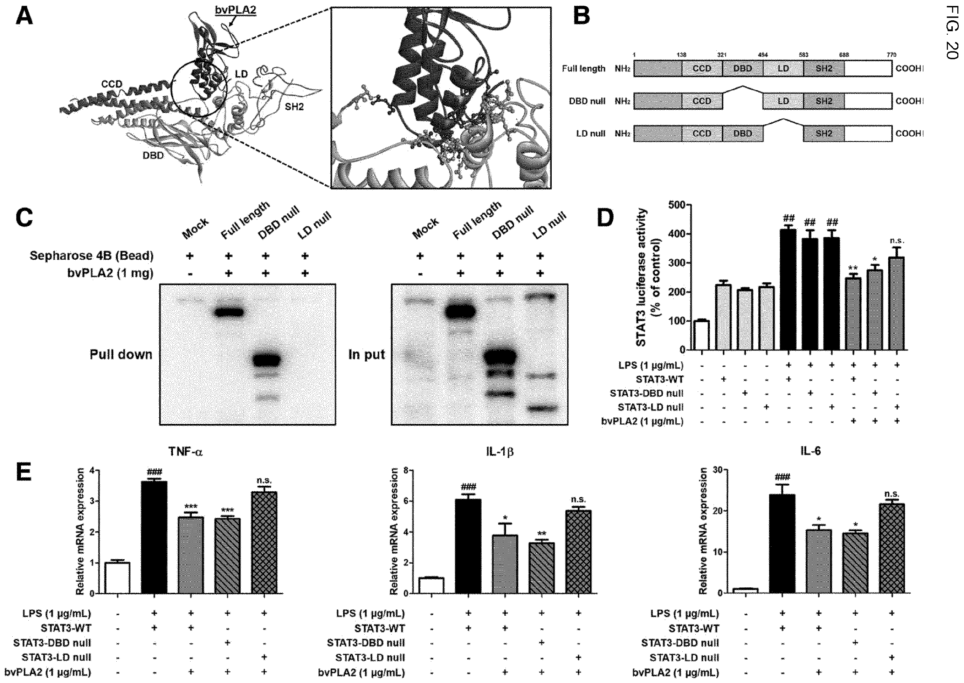

FIG. 20 shows direct binding between the linker domain (LD) of STAT3 and the bee venom extract. FIG. 20A shows an enlarged image of a docking model of bvPLA2 and STAT3 in which the purple part indicates bvPLA2 and the green part indicates STAT3. FIG. 20B shows schematic domain structures of several types of STAT3 recombinant protein, FIG. 20C shows pull-down analysis results, FIG. 20D shows LPS-induced STAT3 luciferase activity analysis results, and FIG. 20E shows results of evaluating the mRNA expression levels of proinflammatory cytokines (TNF-a, IL-1β, and IL-6) in BV-2 cells transfected with several mutant forms of STAT3 by qRT-PCR. Each value represents the mean±SEM of three different experiments. ##p < 0.01, ###p < 0.001, indicates significant differences from a control group, and *p < 0.05, **p < 0.01, ***p < 0.001 indicates significant differences from an LPS-treated wild type group.

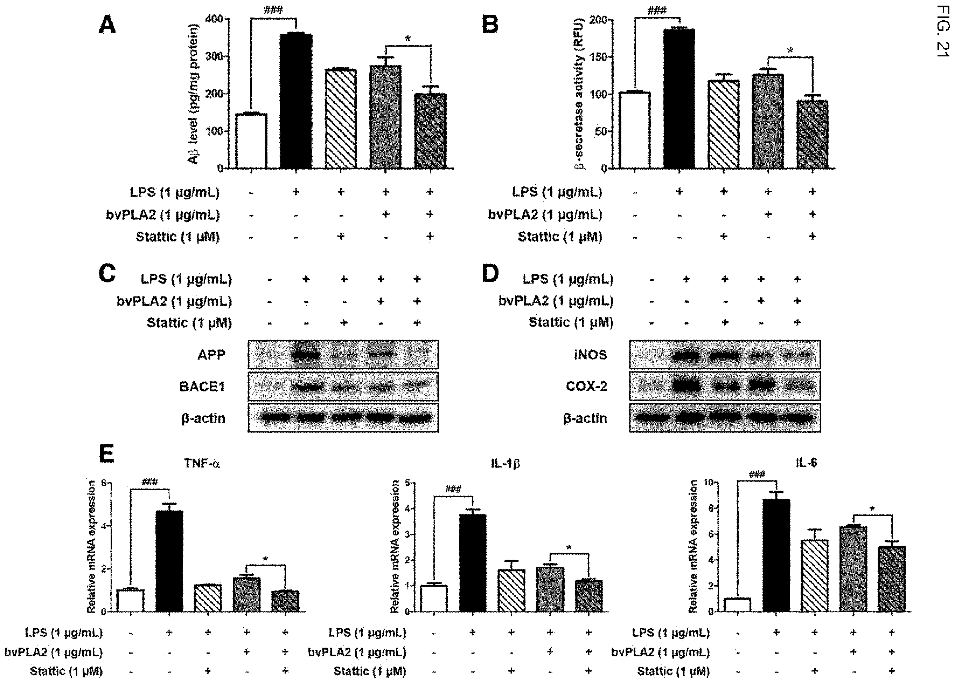

FIG. 21 shows combination effects of the bee venom extract and a STAT3 inhibitor in BV-2 cells. FIG. 21A shows results of evaluating the level of Aβ₁₋₄₂ in BV-2 cells by ELISA, FIG. 21B shows β-secretase activity analysis results in BV-2 cells, FIG. 21C shows Western blot analysis results of APP and BACE1 in BV-2 cells, and FIG. 21D shows Western blot analysis results of iNOS and COX-2 in BV-2 cells. FIG. 21E shows evaluation of mRNA expression levels of pro-inflammatory cytokines (TNF-a, IL-1β, and IL-6) in BV-2 cells treated with bvPLA2 or Stattic by qRT- PCR. Each value represents the mean±SEM of three different experiments. ###p < 0.001 indicates significant differences from a control group, and *p < 0.05 indicates significant differences from the bvPLA2-treated group.

### BEST MODE

Hereinafter, the present invention will be described in detail.

The present inventors have completed the present invention by determining that a bee venom extract containing PLA2 at a specific concentration among bee venom extracts containing various concentrations of phospholipase A2 (PLA2) purified from original bee venom improves treatment efficacy in an animal model of Parkinson's disease and Alzheimer's disease.

As used herein, the term "prevention" refers to any action that inhibits or delays the onset of neuroinflammatory disorders or at least one of symptoms of the disorders by administration of a pharmaceutical composition or a functional food composition according to the present invention. It also includes treatment of a subject in remission of the disorder to prevent or block recurrence is included.

As used herein, the term "treatment" refers to any action to improve or beneficially change, for example, alleviate, reduce, or eliminate, neuroinflammatory disorders or at least one of symptoms of the disorders by administration of the pharmaceutical composition according to the present invention.

As used herein, the term "improvement" refers to any action to improve or beneficially change, for example, alleviate, reduce, or eliminate neuroinflammatory disorders or at least one of symptoms of the disorders by ingestion of the functional food composition according to the present invention.

As used herein, the term "pharmaceutical composition" refers to a composition administered for a specific purpose, which is to be administered to prevent or treat neuroinflammatory disorders or at least one of symptoms of the disorders for the purpose of the present invention.

As used herein, the term "functional food" includes food manufactured and processed using raw materials or ingredients useful for the human body in accordance with Act No. 6727 of the Health Functional Food Act, and refers to foods with high medical and healthcare effects that are processed to efficiently exhibit body modulation functions such as prevention, bio-defense, immunity, and recovery of neuroinflammatory disorders for the purpose of the present invention in addition to nutritional supply.

As used herein, the term "extract" refers to a substance obtained by extracting a component of a natural product regardless of extraction methods, extraction solvents, extracted components, or forms of the extract. It may include any material obtainable after extracting a component of the natural product by processing or treating it by a different method.

The present invention provides a pharmaceutical composition for preventing or treating neuroinflammatory disorders, comprising a bee venom extract as an active ingredient.

As used herein, the "bee venom (BV)" is a mixture of acidic and basic secretions produced in the abdomen of bees (Apis mellifera) and has a colorless, bitter liquid form. It includes melittin, apamin, mast cell degranulating (MCD) peptides, and phospholipase A2 (PLA2) enzymes as main components, and also includes other trace components. It is not limited to being obtained from bees.

The bee venom extract may be a bee venom extract having a phospholipase A2 (PLA2) content of 50 to 85%, preferably a bee venom extract having a PLA2 content of 70 to 80%, more preferably a PLA2 content of 78%.

The bee venom extract may be prepared by extracting from the bee venom according to a method commonly used in the art, and a commercially available one may be purchased and used. However, the present invention is not limited thereto.

The bee venom extract may improve motor function deterioration due to neurotoxicity, and may have neuroprotective effects by reducing polarization of Th1 and Th17 and increasing TH-positive dopaminergic neurons. The bee venom extract may have an effect of alleviating the neuroinflammation by reducing the expression of pro-inflammatory cytokines and activating regulatory T cells to control the neuroinflammatory response. In addition, the bee venom extract reduces activity of beta-secretase (β-secretase), thereby inhibiting accumulation of beta-amyloid (Aβ).

According to an embodiment of the present invention, an effect of improving motor function, a neuroprotective effect, etc. were observed in an animal model of Parkinson's disease administered with a bee venom extract having a PLA2 content of 31%, 53%, or 78%, and in particular, it was determined that the bee venom extract containing 78% of PLA2 showed the greatest effects. More details will be described later in the embodiments below.

The present invention provides a pharmaceutical composition for subcutaneous administration for preventing or treating neuroinflammatory disorders, comprising a bee venom extract as an active ingredient.

The bee venom extract may be a bee venom extract having a phospholipase A2 (PLA2) content of 50 to 85%, preferably a bee venom extract having a PLA2 content of 70 to 80%, more preferably a PLA2 content of 78%.

The bee venom extract may be administered by a subcutaneous route, and may be administered subcutaneously in an amount of 3.75 mg/kg or more and less than 7.5 mg/kg.

The present invention provides a pharmaceutical composition for preventing or treating neuroinflammatory disorders, comprising the bee venom extract as an active ingredient above and one or more inhibitors selected from STAT3 inhibitors and NF-κB inhibitors.

When the pharmaceutical composition is used in combination with an inhibitor that inhibits the activity of STAT3 or NF-κB, the neuroinflammation alleviation effect and the amyloid accumulation inhibitory effect of the bee venom extract can be further improved.

The STAT3 inhibitors may be selected from Stattic, STA-21, S31-201, S31-M2001, S31-1757, curcumin-proline, cryptotanshinone, HIC 1, PD153035, TG101209, PP2, Sophoraflavanone G and the like, and the NF-κB inhibitors may be selected from Bay 11-7802, PDTC, MG-132, MG-115, MLN-4924, JSH-23, PGE2, LY 294002, and the like, but is not limited thereto.

In the pharmaceutical composition according to the present invention, the neuroinflammatory disease may be a disease selected from the group consisting of Alzheimer's disease, vascular dementia, frontotemporal dementia, alcoholic dementia, Parkinson's disease and stroke, but is not limited thereto.

As used herein, the term "neuroinflammatory disorder" refers to inflammatory disorders in the central nervous system mediated by glial cells, and may preferably mean 'cerebral neuroinflammatory disorder', and may include neurodegenerative diseases (degenerative brain disorders), inflammatory brain disorders, metabolic complications, and the like that occur under the influence of neuroinflammation.

The pharmaceutical composition according to the present invention may be prepared according to a conventional method in the pharmaceutical field. The pharmaceutical composition may be combined with an appropriate pharmaceutically acceptable carrier depending on the formulation, and if necessary, it may be prepared by further including excipients, diluents, dispersants, emulsifiers, buffers, stabilizers, binders, disintegrants, solvents, etc. As used herein, the term "pharmaceutically acceptable" means that the composition is not toxic to cells or humans exposed to the composition. The appropriate carrier and the like do not inhibit the activity and properties of the bee venom extract according to the present invention, and may be selected differently depending on the administration type and formulation.

The pharmaceutical composition according to the present invention may be applied in any formulation, and more specifically, it may be formulated as oral formations or non-oral formations such as external preparations, suppositories, and sterile injection solutions according to conventional methods. Preferably, it can be formulated as an injection formulation for intraperitoneal or subcutaneous administration.

Among the oral formulations, solid formulations may be in the form of tablets, pills, powders, granules, capsules, etc., and may be prepared by mixing with at least one or more excipients, for example, starch, calcium carbonate, sucrose, lactose, sorbitol, mannitol, cellulose, and gelatin. In addition to simple excipients, lubricants such as magnesium stearate and talc may be included. Further, the capsule formulation may further include a liquid carrier such as fatty oil in addition to the above-mentioned substances.

Among the oral formulations, liquid formulations include suspensions, solutions, emulsions, syrups, etc. In addition to water and liquid paraffin, which are commonly used as simple diluents, various excipients, for example, wetting agents, sweeteners, fragrances, and preservatives, may be included.

The non-oral formulations may include a sterile aqueous solution, a non-aqueous solution, a suspension, an emulsion, a freeze-dried formulation, and a suppository. Non-aqueous solvents and suspending agents may include propylene glycol, polyethylene glycol, vegetable oils such as olive oil, and injectable esters such as ethyl oleate. As the base of the suppository, witepsol, macrogol, Tween 61, cacao butter, laurin fat, glycerogelatin, and the like may be used. Without being limited thereto, any suitable agent known in the art may be used.

In addition, the pharmaceutical composition according to the present invention may further add calcium, vitamin D₃, or the like to enhance therapeutic efficacy.

In the pharmaceutical composition according to the present invention, the pharmaceutical composition may be administered in a pharmaceutically effective amount. As used herein, the term "pharmaceutically effective amount" means an amount sufficient to treat a disease at a reasonable benefit/risk ratio applicable to medical treatment and not to cause side effects.

The effective dose level of the pharmaceutical composition is determined differently depending on factors including its intended use, age, sex, body weight and health status of the patient, the type of disease, severity, drug activity, sensitivity to drug, administration method, administration time, administration route and excretion rate, treatment duration, and drugs used in combination or concurrently, and other factors well known in the medical field. For example, although not constant, generally 0.001 to 100 mg/kg, preferably 0.01 to 10 mg/kg, may be administered once to several times a day. The above dosage does not limit the scope of the present invention in any way.

The pharmaceutical composition according to the present invention may be administered to any animal capable of developing a neuroinflammatory disease, and the animal may include, for example, not only humans and primates, but also livestock such as cattle, pigs, horses, and dogs.

The pharmaceutical composition according to the present invention may be administered by an appropriate administration route depending on the formulation, and may be administered through various routes, either oral or non-oral, as long as it can reach the target tissue. It may be administered by a conventional administration method which includes, but not particularly limited to, for example, oral, rectal or intravenous, muscle, skin application, respiratory inhalation, or intrauterine epidural or intracere-broventricular injection.

Preferably, it may be administered by a subcutaneous route, and in this case, the bee venom extract may be administered in an amount of 3.75 mg/kg or more and less than 7.5 mg/kg.

The pharmaceutical composition according to the present invention may be used alone or in combination with surgery or other drug treatment for the preventing or treating neuroinflammatory disorders.

The present invention provides a method for subcutaneous administration of the pharmaceutical composition for preventing or treating neuroinflammatory disorders.

According to one embodiment of the present invention, when the bee venom extract was administered by the intraperitoneal route or the subcutaneous route, it was determined that the therapeutic effect of the neuroinflammatory disorder such as Parkinson's disease or Alzheimer's disease was higher when administered by the subcutaneous route. More details will be described later in the embodiments below.

The present invention provides a functional food composition for preventing or treating neuroinflammatory disorders, comprising the bee venom extract as an active ingredient.

The bee venom extract may be a bee venom extract having a phospholipase A2 (PLA2) content of 50 to 85%, preferably bee venom extract having a PLA2 content of 70 to 80%, more preferably a PLA2 content of 78%.

The present invention provides a functional food composition for preventing or treating neuroinflammatory disorders comprising the above functional food composition and one or more inhibitors selected from and STAT3 inhibitors and NF-κB inhibitors.

Corresponding features may be substituted for the above-mentioned parts.

For the functional food composition according to the present invention, the functional food may be prepared as a powder, granules, tablets, capsules, syrups, or beverages for the purpose for preventing or treating neuroinflammatory disorders. There is no limitation in the form that the functional food can take, and it can be formulated in the same manner as the pharmaceutical composition, and used as a functional food or added to various foods.

For the functional food composition according to the present invention, the functional food may include all food in a conventional sense. For example, it may be beverages and various drinks, fruits and their processed foods (canned fruit, jam, etc.), fish, meat and their processed foods (ham, bacon, etc.), breads and noodles, cookies and snacks, and dairy products (butter, cheese, etc.), and may include all functional foods in a conventional sense. It may also include food used as feed for animals.

The functional food composition according to the present invention may be prepared by further including pharmaceutically acceptable food additives and other suitable auxiliary ingredients commonly used in the art. Whether or not it is suitable as a food additive can be judged by the standards and criteria in accordance with the general rules and general test methods of the Food Additives Codex approved by the Food and Drug Administration for the relevant item, unless otherwise specified. The items listed in the 'Food Additives Codex' include, for example, chemical compounds such as ketones, glycine, calcium citrate, nicotinic acid, and cinnamic acid; natural additives such as persimmon pigment, licorice extract, crystalline cellulose, high pigment, and guar gum; mixed preparations such as a sodium L-glutamate preparation, a noodle-added alkali agent, a preservative preparation, and a tar dye preparation, etc.

The other auxiliary ingredients may further contain, for example, flavoring agents, natural carbohydrates, sweeteners, vitamins, electrolytes, colorants, pectic acid, alginic acid, organic acids, protective colloidal thickeners, pH adjusters, stabilizers, preservatives, glycerin, alcohols, and carbonation agents. In particular, as the natural carbohydrate, monosaccharides such as glucose and fructose, disaccharides such as maltose and sucrose, polysaccharides such as dextrin and cyclodextrin, and sugar alcohols such as xylitol, sorbitol, and erythritol may be used, and as the sweetener, natural sweeteners such as taumatine and stevia extract or synthetic sweeteners such as saccharin and aspartame may be used.

For example, a functional food in tablet form may be granulated by a conventional method by mixing a mixture of the bee venom extract, which is the active ingredient of the present invention, with excipients, binders, disintegrants, and other additives, followed by compression molding with lubricants, etc. Alternatively, the mixture may be directly compression molded. In addition, the functional food in the form of tablets may contain a flavor enhancer or the like, if necessary.

Among functional foods in the form of capsules, hard capsules can be prepared by filling a mixture of the bee venom extract with additives such as excipients in common hard capsules. Soft capsules can be prepared by filling a mixture of the bee venom extract with additives such as excipients in a capsule base such as gelatin. The soft capsules may contain a plasticizer such as glycerin or sorbitol, a colorant, a preservative, and the like, if necessary.

A functional food in the form of a pill can be prepared by molding a mixture of the bee venom extract with an excipient, a binder, a disintegrant, etc. using a known method. It may be coated with sucrose or other coating agent, if necessary, or its surface may be coated with a material such as starch or talc.

A functional food in the form of granules can be prepared in granular form of a mixture of the bee venom extract and excipients, binders, disintegrants, etc., by a conventionally known method and if necessary, may contain flavoring agents or flavor enhancers.

The effective dose of the bee venom extract contained in the functional food according to the present invention may be appropriately adjusted depending on the purpose of use, such as prevention or improvement of neuroinflammatory disorders.

The functional food composition is advantageous in that there are no side effects which may occur during long-term administration of general drugs because it uses food as a raw material, has excellent portability, and can be taken as an adjuvant for preventing or improving neuroinflammatory disorders.

### MODES FOR CARRYING OUT INVENTION

Hereinafter, to help the understanding of the present invention, the embodiments will be described in detail. However, the following embodiments are merely illustrative of the present invention, and the scope of the present invention is not limited to the following embodiments. The embodiments of the present invention are provided to explain more completely the present invention to those of ordinary skill in the art.

### <Example 1> Single-dose toxicity evaluation according to the PLA2 content in the bee venom extract in rodents

### 1. Toxicity evaluation method: [C57BL/6N WT + PLA2]

- Animal model: wild type, 7 weeks old, male C57BL/6N mouse (purchased from Daehan Biolink)
- Drug types: a bee venom extract containing 100% of PLA2 (100% PLA2), a bee venom extract containing 80% of PLA2 (80% PLA2), a bee venom extract containing 50% of PLA2 (50% PLA2), a bee venom extract containing 30 % of PLA2 (30% PLA2)
- Route of administration: Subcutaneous injection (SC)
- Dosage: 15mg/kg, 7.5mg/kg, 3.75mg/kg once/day
- Observation period and item: Observation of death daily for 3 days after administration
- Groups: total 65 animals, 5 animals each group below

| | | | |
|---|---|---|---|
| a. | Control (saline, WT) | h. | 50% PLA2 (15mg/kg) |
| b. | 100% PLA2 (15mg/kg) | i. | 50% PLA2 (7.5mg/kg) |
| c. | 100% PLA2 (7.5mg/kg) | j. | 50% PLA2 (3.75mg/kg) |

| | | | |
|---|---|---|---|
| d. | 100% PLA2 (3.75mg/kg) | k. | 30% PLA2 (15mg/kg) |
| e. | 80% PLA2 (15mg/kg) | I. | 30% PLA2 (7.5mg/kg) |
| f. | 80% PLA2 (7.5mg/kg) | m. | 30% PLA2 (3.75mg/kg) |
| g. | 80% PLA2 (3.75mg/kg) | | |

### 2. Evaluation results

Table 1 below shows the results of toxicity evaluation according to Example 1 of the present invention.

Referring to Table 1, it was determined that the approximate lethal dose (ALD) of the bee venom extract containing 30% of PLA2 and the bee venom extract containing 50% of PLA2 was 15 mg/kg, the approximate lethal dose of the bee venom extract containing 80% of PLA2 was 7.5 mg/kg, and the approximate lethal dose of bee venom extract containing 100% of PLA2 is about 3.75 mg/kg.

**[Table 1]**

| PLA2 contents (% in bee venom extract) | Concentration | Mouse survival rate (n/5) |
|---|---|---|
| 100% | 15 mg/kg | 0/5 |
| | 7.5 mg/kg | 0/5 |
| | 3.75 mg/kg | 1/5 |
| 80% | 15 mg/kg | 0/5 |
| | 7.5 mg/kg | 1/5 |
| | 3.75 mg/kg | 5/5 |
| 50% | 15 mg/kg | 0/5 |
| | 7.5 mg/kg | 5/5 |
| | 3.75 mg/kg | 5/5 |
| 30% | 15 mg/kg | 1/5 |
| | 7.5 mg/kg | 5/5 |
| | 3.75 mg/kg | 5/5 |
| 1XPBS (0mg/kg) | | 5/5 |

### <Example 2> Evaluation of therapeutic effects according to the PLA2 content in the bee venom extract in an animal model of Parkinson's disease (PD)

In this example, a test was performed to evaluate the efficacy according to the PLA2 content in bee venom extracts in an animal model of Parkinson's disease that induced by 1-methyl-4-phenyl-1,2,3,6-tetrahydropyridine hydrochloride (MPTP).

### 1. Test substance

### 1) Test substance

- Name: crude Bee Venom (sugar-fed bee venom extract) / bee venom extract containing 31% of PLA2 (PLA2 31%) / bee venom extract containing 53% of PLA2 (PLA2 53%) / bee venom extract containing 78% of PLA2 (PLA2 78%)
- Appearance: White powder
- Amount: 15mg / 11.1mg / 10.3mg / 19.9mg
- Storage conditions: Frozen (-20°C)
- Source: INIST ST Co., Ltd.

### 2) Control material

- Name: Bee Venom Phospholipase A2 (Sigma PLA2, Sigma code #: P9279)
- Appearance: Lyophilized powder
- Amount: 1mg
- Storage conditions: Frozen (-20°C)
- Source: Sigma (Sigma, St. Louis, MO, USA)

### 3) Inducing substance

- Name: MPTP (1-Methyl-4-phenyl-1,2,3,6-tetrahydropyridine hydrochloride)
- Appearance: white powder
- Amount: 100mg
- Storage conditions: room temperature
- Source: Sigma (Sigma, St. Louis, MO, USA)

### 2. Materials and Methods

### 1) Test system

In carrying out the experiment, the mouse is small in size and easy to handle so that it is easy to use in animal experiments, and sufficient tissue results and experimental results can be obtained even using it. 7 week-old male C57BL/6J mice were used for testing, which were supplied from Daehan Biolink (227, Duckho-ro, Samsung-myeon, Eumsung-gun, Chungcheonbuk-do, Korea). This strain of mice is widely used in oncology, immunology, and toxicology studies.
- Date of acquisition: March 02, 2018
- Gender, number of animals and weight range at the time of acquisition: 43 males, 20-25g
- Weight range at start of dosing: 20-25g
- Number of animals used: 43 males

### 2) Breeding environment

### (1) Environmental conditions and breeding methods

Raised under SPF (Specific Pathogen Free) environment, adjusting temperature of 22±1°C, relative humidity of 55±15%, number of ventilation 10-20 times/hr, lighting time of 12 hours (lights on at 7am - lights out at 7pm), and Illuminance 150-300 Lux. All experiments were conducted in accordance with the approval of the Animal Experimental Ethics Committee of Kyunghee University (KHUASP(SE)-17-149).

### (2) Feed and water

Solid feed for laboratory animals was supplied from Orient Bio and freely ingested by the mice, and water was freely ingested using a water bottle after sterilizing water and sewage with a microfilter.

### 3) Test group composition and dosage setting

### (1) Test group composition

- Control group
- MPTP group (no treat group)
- Sigma PLA2 group (0.5mg/kg)
- Crude Bee Venom group (0.5mg/kg)
- PLA2 31% group (bee venom extract containing 31% of PLA2, 0.5mg/kg)
- PLA2 53% group (bee venom extract containing 53% of PLA2, 0.5mg/kg)
- PLA2 78% group (bee venom extract containing 78% of PLA2, 0.5mg/kg)

### (2) Dosage setting

The MPTP group was injected 4 times by intraperitoneal (IP) route 4 times a day at an interval of 2 hours at 20 mg/kg.

Sigma PLA2 group, crude Bee Venom group, PLA2 31% group, PLA2 53% group, and PLA2 78% group were injected subcutaneously (SC) once a day for 6 days at 0.5mg/kg.

### (3) Group separation

Group separation of animals was performed as follows. After MPTP injection, the animals were trained in the behavioral test (Pole test) in the morning of the next day. By the results, the animals were randomly distributed to each group so as to be distributed as evenly as possible by the results and to meet the number specified in the test group composition table.

### (4) identification

Individual identification was distinguished by marking on the tail with an oil-based pen. An individual identification card was attached to each mouse cage with the test number, the name of a chief manager in charge of the test, the name of a manager in charge of the test, and emergency contact information.

### (5) Preparation of administration substance

MPTP 100mg was dissolved in 1 ×free base saline buffer to prepare a concentration of 2.5mg/mL. The concentration value is a value calculated when 200 µL is administered intraperitoneally (IP) at a dosage of 20 mg/kg per mouse.

Sigma PLA2, crude Bee Venom, a bee venom extract containing 31% of PLA2 (PLA2 31%), a bee venom extract containing 53% of PLA2 (PLA2 53%), and a bee venom extract containing 78% of PLA2 (PLA2 78%) were also added to 1×free base saline buffer. They were dissolved in the buffer and prepared to a concentration of 1 mg/mL, diluted to an administration concentration of 0.5 mg/kg, and administered to experimental animals by subcutaneous (SC) route.

### 4) Test method

### (1) Animal testing

In the case of inducing Parkinson's disease with MPTP, the mice were intraperitoneally administered with MPTP-HCI (20 mg/kg free base in saline) 4 times at 2 hour intervals. After 12 hours from the last MPTP injection, Sigma PLA2, crude Bee Venom, PLA2 31%, PLA2 53%, and PLA2 78% (0.5 mg/kg each) were injected subcutaneously for 6 days in mice receiving MPTP (FIG. 1). Some mice were injected with vehicle only as a control group. The mortality rate of mice after MPTP injection was less than about 0-30% in each group. There was no significant difference between each group.

### (2) Measurement of behavioral experiments by pole test

A mouse was placed on the top of a gauze-wrapped wooden pole (length 50 cm, diameter 0.8 cm) with the mouse facing upward (the mouse can go down to the bottom of the pole), the time taken for the mouse to turn completely downward (T-turn) and the total time taken for the mouse to reach the floor (locomotion activity time, T-LA) were recorded, respectively, and the time limit was set to 30 seconds. Each mouse was tested 5 times, and the average of the three best measurements was calculated. Mouse jumping or sliding on the floor was excluded.

### (3) flow cytometry analysis of Th1, Th17, and Treg populations

Flow cytometry analysis of T-helper cell subsets was performed by using fluorescein isothiocyanate (FITC)-conjugated anti-mouse CD4, phycoerythrin (PE)-conjugated anti-mouse CD25, Alexa Fluor 647 anti-mouse/rat Foxp3, PE-conjugated anti-mouse IFN-y, and PerCP-Cyanine5.5-conjugated anti-mouse/rat IL-17A antibodies.

To stain regulatory T cells (Tregs), spleen cells at a concentration of 2×10⁶ cells/mL were collected, washed twice with a PBS buffer, and then stained with FITC-conjugated anti-CD4 and PE-labeled anti-CD25 antibodies diluted in a staining buffer for 30 min in the dark at 4°C. Then, the cells were washed twice with PBS and fixed in the dark at 4°C in a fixation/permeabilization buffer for 1 hour. Subsequently, cells were washed twice with PBS and stained with Alexa Fluor 647 anti-Foxp3 antibody overnight in the dark at 4°C. After washing, the cells were fixed in 1% paraformaldehyde and stored in the dark at 4°C for subsequent detection.

For intracellular cytokine staining, splenocytes at a concentration of 2×10⁶ cells/mL were stimulated with 50 ng/mL of phorbol myristate acetate (PMA) and 1,000 ng/mL of ionomycin. After stimulation for one hour, GolgiStop was added and the cells were cultured for 4 hours. The cultured cells were collected, washed twice with PBS, and then stained with FITC-labeled anti-CD4 antibody diluted in the staining buffer in the dark at 4°C for 30 minutes. After washing with PBS, the cells were fixed in an I.C fixation buffer for 30 min in the dark at room temperature. After washing with PBS, cells were stained with PE-conjugated anti-IFN-y and PerCP-Cyanine5.5-conjugated anti-IL-17A antibodies overnight in the dark at 4°C. After washing with PBS, cells were fixed in 1% paraformaldehyde and stored in the dark at 4°C. Then, detection was performed. Samples were analyzed with a FACS Calibur flow cytometer and then data was generated in graphical and tabular formats by using Flowjo software.

### (4) Tissue processing and immunohistochemistry (IHC)

Mice were anesthetized with isoflurane (Forane solution; Choongwae Pharmaceutical, Seoul, Korea), and PBS was injected at the end of the heart for perfusion, followed by perfusion with 4% paraformaldehyde dissolved in 0.1M phosphate buffer for fixation.

The brain of the fixed mice were dissected, fixed with 4% paraformaldehyde at 4°C for two days, and then transferred to a 30% sucrose solution and stored at 4°C until it subsides. Tissues were fixed with OCT compounds and successively cut into 30 µm-thick coronal sections in a cryostat using a sliding microtome. All six consecutive tissues were collected and processed for immunohistochemical staining.

Tyrosine hydroxylase (TH; 1:1000) was attached as a primary antibody. After washing with PBS, a biotinylated secondary antibody was attached to the tissue and the tissue was treated with an avidin-biotin complex kit (Vectastain ABC kit). Then, the tissue was colored with 0.05% diaminobenzidine-HCI (DAB) and 0.003% hydrogen peroxide dissolved in a 0.1 M phosphate buffer as reaction materials. Then, the colored tissue was mounted with a slide glass and a cover glass, and analyzed with an optical microscope.

### (5) Stereological cell counting

An objective stereoscopic assessment of the total number of TH-positive dopaminergic neurons in the subtantia nigra (SN) region of the brain was performed using an optical fractionator method in the Olympus computer-assisted stereotype toolbox (CAST) system version 2.1.4.

For cell counting, the entire SN region was used from the tip of the pars compacta to the tip of the tail of the pars reticulate (anteroposterior, -2.06 mm to -4.16 mm from bregma). Actual calculations were performed using a 100x magnification (Oil objective). Total cell number was calculated according to the optical fractionator equation. More than 300 points were analyzed for all tissues of each specimen.

### (6) Statistical analysis

For analysis of significance differences for all experimental results, the average value was analyzed with variance in Graphpad Prism software (Version 5, CA, USA). In a multiple comparison analysis, in the case that the P-value was less than 0.05 according to the one-way Anova and Newman-Kelus test, it was determined to exhibit a statistically significant difference. In a single comparison, in the case that the P-value was less than 0.05 according to two-tailed Student's t-test, it was determined to exhibit a statistically significant difference. All experiments were performed in a blind manner and repeated independently under identical conditions.

### 3. Results

### 1) Determination of effects according to PLA2 content in bee venom extract on motor function in MPTP-induced neurotoxicity

To evaluate the effect according to PLA2 content in the bee venom extract for MPTP-induced neurotoxicity, MPTP was administered to the mice, and Sigma PLA2, crude Bee Venom, PLA2 31% (bee venom extract containing 31% of PLA2), PLA2 53% (bee venom extract containing 53% of PLA2), PLA2 78% (bee venom extract containing 78% of PLA2), or physiological saline was administered subcutaneously to the mice once a day for 6 days from 12 hours after the last MPTP injection.

Various behavioral tests, including locomotor activity, a rotarod test, a forepaw adjusting step, and the pole test, are commonly used in Parkinson's disease (PD) mouse models. In this example, the neuroprotective effect of the bee venom extracts on PD-related movement disorders was evaluated by the pole test in an MPTP-induced mouse model.

As a result, as shown in FIG. 2, the group administered with only MPTP significantly extended the time of T-turn and T-LA compared to other controls. However, the PLA2 31%, PLA2 53%, PLA2 78% groups significantly shortened the time of T-turn and T-LA as much as the group administered with sigma PLA2, and it was proven that these groups had an improvement effect. In particular, the group administered with PLA2 78% showed the greatest effect. On the other hand, there was no significant difference in the T-turn time and the T-LA time between the MPTP group and the crude Bee Venom group.

### 2) Determination of effect according to PLA2 content in bee venom extract on population of Treg cells in MPTP-induced PD mice

In previous studies, PD patients had a decreased proportion of Treg cells compared to controls, and a decreased ability to inhibit the effector T cell function was observed from PD patients. Accordingly, the effect of the content of the bvPLA2 component on the proportion of Treg cells was evaluated by flow cytometric analysis.

As a result, as shown in FIG. 3, no significant difference was found in the populations of CD4+ CD25+ Foxp3+ Treg cells between the control group and the MPTP group. However, it was determined that the population of Treg cells increased by more than 70% in the group administered with Sigma PLA2 and PLA2 78% compared to the control group, and the population of Treg cells increased slightly in the group administered with PLA2 31% and PLA2 53%. On the other hand, there was no significant difference in the population of Treg cells in the group administered with crude Bee Venom.

### 3) Determination of effect according to PLA2 content in bee venom extract on Th1 and Th17 cell counts in MPTP-induced PD mice

Next, the effect according to the content of PLA2 in the bee venom extracts on the T-helper cell phenotype was determined. Expression of IFN-y and IL-17A was substituted for Th1 and Th17 cells, and the distribution of these cells was observed by flow cytometry.

As a result, as shown in FIG. 4, the population of Th1 was increased, and the Th1/Th2 balance was shifted toward Th1 in the MPTP group, showing that the Th1-type response was improved. It was also determined that Th17 cells increased in the MPTP group. On the other hand, IFN-y and IL-17A expressions of polarized Th1 and Th17 cells were decreased in the groups administered with Sigma PLA2 and PLA2 78% compared to the MPTP group.

### 4) Determination of effect according to PLA2 content in bee venom extract on SN dopaminergic neurons on MPTP neurotoxicity

To evaluate the protective effect of the content of PLA2 in the bee venom extracts on dopaminergic neurons, the number of TH (tyrosine hydroxylase)-positive neurons was measured.

As a result, as shown in FIG. 5, the MPTP group showed a 3-fold decrease in the number of TH-positive neurons compared to the control group. As a result of analyzing the dopaminergic neurons surviving in TH-immunostained striatum after treatment with MPTP and the bee venom extracts, it was determined that the number of surviving TH-positive neurons in the SN was increased in the groups administered subcutaneously with Sigma PLA2 and PLA2 78%. Further, it was determined that the number of TH-positive neurons was increased moderately in the groups administered with PLA2 31% and PLA2 53%. On the other hand, for the group administered with crude Bee Venom, dopaminergic neurons in the SN were significantly reduced.

### 4. Conclusion

In this example, the maximum efficacy according to the content of PLA2 in the bee venom extracts was investigated in the animal model of Parkinson's disease (PD). In the previous study results, it was determined that the best route of administration of the bee venom extract to prevent transient progression of PD pathology was subcutaneous (SC), and in the case that the bee venom extract was injected subcutaneously, dopaminergic neurons were protected by modulating the neuroinflammatory response in the PD mouse model induced by MPTP.

According to this example confirming the optimal content of the PLA2 component in the bee venom extract to improve the pathological aspect of PD, in the case of treating with a drug containing 78% of PLA2, it was confirmed that the MPTP toxicity model exhibited the best neuroprotective effect including improved motor function at a level similar to that of sigma PLA2, reduction of Th1 and Th17 polarization, and increase of TH-positive dopaminergic neurons.

Therefore, in the MPTP-induced PD mouse model, the drug containing the bee venom extract containing PLA2 78% (PLA2 78%), which is an optimal PLA2 component content for preventing nerve damage, appears to be the most effective compared to the drug containing the bee venom extract containing 31% or 53% of PLA2, and the subcutaneous administration route of the PLA2 78% drug is considered to have potential applications in treatment of PD patients.

### <Example 3> Evaluation of therapeutic effect according to the content of PLA2 component in bee venom extract in animal model of Alzheimer's disease (AD)

### 1. Experimental method

### 1) material

bvPLA2 (bee venom extract containing 75-85% of PLA2) was supplied from INISTst Co., LTD, Gyeonggi-do, Republic of Korea. It was dissolved in phosphate-buffered saline (PBS; final concentration 1 mg/mL) and stored at -20°C until use.

LPS was purchased from Sigma (serotype 0111:B4; Sigma, St. Louis, MO, USA). LPS (final concentration 1 mg/mL) was dissolved in PBS, and aliquots thereof were stored at -20°C until use..

Ap₁₋₄₂ was purchased from Tocris Bioscience (rat; Bristol, United Kingdom), and Ap₁₋₄₂ (final concentration 200 µM) was dissolved in 0.1% ammonia and stored at -70°C.

### 2) Water maze test

A water maze experiment was performed according to a method commonly used for memory tests. The maze experiment was performed by the SMART-CS (Panlab, Barcelona, Spain) program and equipment. A circular plastic pool (height: 35 cm, diameter: 100 cm) was filled with water prepared opaquely with skim milk maintained at 22-25 °C. An escape platform (height: 14.5 cm, diameter: 4.5 cm) is submerged 1-1.5 cm below the water level. The test was performed on a single platform at two rotational starting positions. After the test, the mice stayed on the platform for 120 seconds and then sent to their cages. The escape latency and escape distance of each mouse were monitored with a camera above the center of the pool connected to the SMART-LD program (Panlab, Barcelona, Spain).

### 3) Probe test

To test memory retention, a probe test was performed 24 hours after the water maze experiment. The platform was removed from the pool used in the water maze experiment, and the mice were allowed to swim freely. The swimming pattern of each mouse was monitored and recorded for 60 seconds using the SMART-LD program (Panlab, Barcelona, Spain). Retained spatial memory was calculated by the time spent in the target quadrant area.

### 4) Passive avoidance performance test

A passive avoidance experiment is generally known as a simple experiment for testing memory. The passive avoidance response is determined using a "step- through" apparatus (Med Associates Inc., Vermont, USA) that is divided into an illuminated compartment and a dark compartment (20.3x15.9x21.3 cm, respectively) adjacent to each other via a small gate with a grid bottom of 3.175 mm stainless steel rods spaced 8 mm apart.

On the first day, mice were placed in the illuminated compartment away from the dark compartment for training trials. When the mice moved completely into the dark compartment, an electric shock (0.45 mA, 3s duration) was generated. After that, the mice were returned to their cages. One day after the training test, mice were placed in the illuminated compartment and the retention time to enter the dark compartment defined as "retention" was measured. The time taken for the mouse to enter the dark compartment was recorded and described by step-by-step retention time. The memory test set a three-minute deadline.

### 5) Collection and preservation of brain tissue

After behavioral experiments, mice were perfused with PBS containing heparin under anesthesia by CO₂ inhalation. After the skull was immediately removed from the brain, only the hippocampus region was isolated and stored at -80°C until biochemical analysis.

### 6) Thioflavin S staining

After being transferred to a 30% sucrose solution, the brain was cut into 20 µm sections using a cryostat microtome (Leica CM 1850; Leica Microsystems, Seoul, Korea). After washing with distilled water for 5 min, the brain sections were transferred to gelatin-coated slides and placed in 50% ethanol containing 1% thioflavin S (Sigma, St Louis, MO, USA) for 8 min. Then, the brain sections were washed with distilled water and then dehydrated for 2 min in each grade of ethanol of 50, 70, 90, and 100%. The sections were then mounted with a mounting medium (Fluoromount^{™} Aqueous Mounting Medium; Sigma, St. Louis, MO, USA). Thioflavin S staining was observed using a fluorescence microscope (Axio Observer A1; Carl Zeiss, Oberkochen, Germany) (×50 and ×200).

### 7) β-secretase activity assay

β-secretase activity in the mouse brain was determined using a commercially available β-secretase activity assay kit (Abcam, Inc., Cambridge, MA, USA). A solubilized membrane was extracted from brain tissues using a β-secretase extraction buffer, incubated on ice for 1 hour, and centrifuged at 5000xg for 10 minutes at 4° C to collect supernatant.

A total of 50 µL of sample (100 µg of total protein) or blank (50 µL of β-secretase extraction buffer) was added to each well (96-well plate), then 50 µL of 2× reaction buffer and 2 µL of β-secretase substrate were incubated for 1 hour in a dark room at 37°C. Fluorescence was read at excitation and emission wavelengths of 335 and 495 nm, respectively, using a fluorescence spectrometer (Gemini EM; Molecular Devices, CA, USA).

### 8) Measurement of β-amyloid (Aβ)

Lysates of brain tissue were obtained through a protein extraction buffer containing protease inhibitors. Ap₁₋₄₂ and Aβ₁₋₄₀ levels were measured using the specific mouse β-amyloid peptide 1-42 enzyme-linked immunosorbent assay (ELISA) kit (CSB-E10787m; CUSABIO, Houston, USA) and mouse β-amyloid peptide 1-40 ELISA kit (CSB-E08300m; CUSABIO, Houston, USA), respectively.

Proteins were extracted from brain tissues using a protein extraction buffer (PRO-PREP; Intron Biotechnology, Kyungki-do, Korea), incubated on ice for 1 hour, and centrifuged at 13,000xg for 15 minutes at 4°C. Briefly, 100 µL of the sample was added to a precoated plate and incubated at 37° C for 2 h. After removing unbound material, a biotin-conjugated antibody specific for Aβ was added to the wells. After washing, avidin-conjugated horseradish peroxidase (HRP) was added to the wells. After washing to remove unbound avidin-enzyme reagent, a substrate solution was added to the wells and developed in proportion to the amount of bound Aβ in the initial step. Color development was stopped and color intensity was measured.

### 9) Immunohistochemical staining

After being transferred to a 30% sucrose solution, the brain was cut into 20 µm sections using a cryostat microtome (Leica CM 1850; Leica Microsystems, Seoul, Korea). After washing twice in PBS (pH 7.4) for 10 min each, the samples were incubated with PBS containing 3% hydrogen peroxide and 1 % Triton-X (Triton-X) for 30 minutes for the purpose of antigen retrieval and blockage of endogenous peroxidase, and additionally washed twice with PBS for 10 minutes each.

The brain sections were blocked in 3% bovine serum albumin (BSA) solution for 1 hour, and then incubated with glial fibrillary acidic protein (GFAP; 1:300; Santa Cruz Biotechnology, Inc., Santa Cruz, CA, USA), inducible nitric oxide synthase (iNOS; 1:300; Abcam, Inc., Cambridge, MA, USA), ionized calcium binding adapter molecule 1 (IBA-1; 1:300; Abcam, Inc., Cambridge, MA, USA), and cyclooxygenase 2 (COX-2; 1:300, Novus Biologicals, Inc., CO, USA) at 4 °C overnight.

After incubation with the primary antibody, the brain sections were washed three times with PBS for 10 min each. After washing, the brain sections were incubated with biotinylated goat anti-rabbit, goat anti-mouse, or donkey anti-goat IgG-horseradish peroxidase (HRP) secondary antibody (1:500; Santa Cruz Biotechnology, Inc., Santa Cruz, CA, USA) at room temperature for 1-2 hours. The brain sections were washed three times with PBS for 10 min each and visualized by chromogen diaminobenzidine (Vector Laboratories, Burlingame, CA, USA) reaction for up to 10 min.

Finally, the brain sections were dehydrated in ethanol, removed in xylene, mounted on Permount (Fisher Scientific, Hampton, NH, USA), and evaluated by an optical microscope (Microscope Axio Imager. A2; Carl Zeiss, Oberkochen, Germany; x50 and x200).

### 10) Western blot analysis

To detect target proteins, specific antibodies to iNOS, IBA-1, GFAP, APP and BACE1 (1:1000; Abcam, Inc., Cambridge, UK), COX-2 (1:1000; Novus Biologicals, Inc., CO, USA), c-Jun N-terminal kinase (JNK), extracellular signal-regulated kinase (ERK) 1/2, p-p38 and p38 (1:000; Cell signaling Technology, Inc., MA, USA), p-STAT3, STAT3, p-ERK1/2, p-JNK, and β-actin (1:200; Santa Cruz Biotechnology Inc., Santa Cruz, CA, USA) were used.

Blots were incubated with corresponding conjugated secondary antibodies such as anti-mouse, anti-rabbit and anti-goat obtained from Santa Cruz Biotechnology Inc.(Santa Cruz, CA, USA). Immunoreactive proteins were detected with an enhanced chemiluminescence Western blotting detection system.

### 11) Cytokine level measurement

Pro-inflammatory and anti-inflammatory cytokine levels were measured by quantitative reverse transcription polymerase chain reaction (qRT-PCR). Total RNA was extracted from hippocampal tissue using RiboEX (Geneall biotechnology, Seoul, Korea), and cDNA was synthesized using a High-Capacity cDNA Reverse Transcription kit (Thermo Scientific, Waltham, MA, USA).

qRT-PCR was performed on a 7500 real-time PCR system (Applied Biosystems, Foster City, CA, USA) for custom designed primers, and β-actin was used as a housekeeping control using a HiPi Real-Time PCR SYBR green master mix (ELPIS biotech, Daejeon, Korea).

A cycling condition consisting of an initial denaturation step of 3 minutes at 94°C, a denaturation step of 30 seconds at 94°C, an annealing step of 30 seconds at 60°C, and an extension step of 1 minute at 72°C, was continued until 40 cycles. Values obtained for target gene expression were normalized to β-actin and quantified for expression in control samples.

Each sample was performed with the primers in Table 2 below.

**[Table 2]**

| factor | forward/reverse | primer |
|---|---|---|
| β-actin | forward | 5'- GGCTGTATTCCCCTCCATCG - 3' |
| | reverse | 5'- CCAGTTGGTAACAATGCCATGT - 3' |
| TNF-α | forward | 5'- TCTTCTCATTCCTGCTTGTGG - 3' |
| | reverse | 5'- CACTTGGTGGTTTGCTACGA - 3' |
| IL-1β | forward | 5'- CCTTCCAGGATGAGGACATGA - 3' |
| | reverse | 5'- TGAGTCACAAGAGGATGGGCTC - 3' |
| IL-6 | forward | 5'- GAGGATACCACTCCCAACAGACC - 3' |
| | reverse | 5'- AAGTGCATCATCGTTGTTCATACA - 3' |
| IL-10 | forward | 5'- TCTGAGCCACTCACATCTGC - 3' |
| | reverse | 5'- TCAGGGGAACTGCTAGTGCT - 3' |
| IL-4 | forward | 5'- GGTCTCAACCCCCAGCTAGT - 3' |
| | reverse | 5'- GCCGATGATCTCTCTCAAAGTGAT - 3' |
| TGF-β | forward | 5'- CTCCCGTGGCTTCTAGTGC - 3' |
| | reverse | 5'- GCCTTAGTTTGGACAGGATCTG - 3' |

### 12) BV-2 microglial cells culture

Microglia BV-2 was cultured and treated simultaneously with LPS (1 µg/mL) or Aβ₁₋₄₂ (2.5 µM) and various concentrations of bvPLA2 (0.01, 0.1, 1 µg/mL) dissolved in PBS. Cells were harvested after 24 hours to measure cell viability, Nitric Oxide concentration, and a level of pro-inflammatory cytokines.

### 13) Cell viability analysis

BV-2 cells were plated in 96-well plates and then treated with bvPLA2 (0-1 µg/mL) for 24 hours. After treatment, cell viability was measured by MTT solution [3-(4,5-Dimethylthiazol-2-yl)-2,5-Diphenyltetrazolium Bromide] (Sigma Aldrich, St. Louis, MO). MTT solution with 1/10 volume of cell medium was added to each well, and the mixture was incubated in a CO2 incubator for 2 hours before removal. After removing the mixture from the cells, dimethyl sulfoxide (DMSO) was added to the same volume of the medium. Then, the absorbance of each well was read with a microplate absorbance reader at a wavelength of 570 nm.

### 14) Nitric oxide analysis

BV-2 cells were grown in 96-well plates and then cultured with or without LPS (1 µg/mL) in the absence or presence of various concentrations of bvPLA2 (0.01, 0.1, 1 µg/mL) for 24 hours. The nitrite concentration of the supernatant was analyzed by a nitric oxide detection kit (NO detection kit, Intron Biotechnology, Kyungki-do, Korea). Absorbance at 520 nm was measured by a microplate absorbance reader, and a series of known concentrations of sodium nitrite was used as standard.

### 15) Pull-down assay

bvPLA2 was conjugated with epoxy-activated Sepharose 4B (GE Healthcare Korea, Seoul, Korea).

Briefly, 1 mg of bvPLA2 was dissolved in 1 mL of coupling buffer (0.1 M NaHCO₃ and 0.5 M NaCl, pH 11.0). 0.1 g of epoxy-activated Sepharose 4B beads were swollen and washed with 1 mM HCI on a sintered glass filter, followed by being washed with the coupling buffer. Epoxy-activated Sepharose 4B beads were added to a coupling buffer containing bvPLA2 and spun overnight at 4°C. Control unconjugated Sepharose 4B beads were prepared as above without bvPLA2.

After washing, unoccupied binding sites were blocked with a blocking buffer (0.1M Tris-HCI, pH 8.0) at room temperature for 3 hours. bvPLA2-conjugated Sepharose 4B was washed with 3 cycles of replacement pH wash buffer (buffer 1: 0.1 M acetate and 0.5 M NaCl, pH 4.0; buffer 2: 0.1 M Tris-HCI and 0.5 M NaCl, pH 8.0). Then, the bvPLA2-conjugated beads were equilibrated with a binding buffer (0.05 M Tris-HCI and 0.15 M NaCl, pH 7.5).

To demonstrate binding of bvPLA2 and STAT3, the STAT3 protein was overexpressed via transfection with STAT3 DNA. BV-2 cells were transfected with STAT3 DNA (700 ng/perwell of a six well plate) using Lipofectamine^{®} 3000 (Invitrogen, Waltham, MA, USA) in Opti-MEM according to the manufacturer's protocol. BV-2 cell lysate (1 mg of protein) was mixed with bvPLA2-conjugated Sepharose 4B or unconjugated Sepharose 4B, and cultured overnight at 4°C. After this, the beads were washed three times with TBST.

The binding protein was eluted with a sodium dodecylsulfate (SDS) loading buffer, separated using SDS/polyacrylamide gel electrophoresis, and then subjected to immunoblotting with an antibody to STAT3 (1:200, Santa Cruz Biotechnology, Dallas, TX., USA).

### 16) Luciferase assay

BV-2 cells were plated in 12 well plates (8×10⁴ cells/well) and were transiently transfected with STAT3-Luc (Stratagene, La Jolla, CA, USA) plasmid using Lipofectamine 3000 in Opti-MEM according to manufacturer's specifications (Invitrogen, Carlsbad, CA, USA) for 24 hours. The transfected cells were treated with 1 µg/mL of LPS, and 0.01, 0.1, and 1 µg/mL of bvPLA2, respectively for 24 hours. Luciferase activity was measured using a Dual-Luciferase^{®} Reporter Assay System kit (Promega, Madison, WI, USA) and a luminomete according to the manufacturer's specifications (WinGlow, Bad Wildbad, Germany).

### 17) Docking experiment

Docking studies of bvPLA2 using STAT3 were performed using Autodock VINA (Trott and Olson, 2010). The three-dimensional structures of STAT3 [PDB: 3CWG] and bvPLA2 [PDB: 1POC] were retrieved from the Protein Data Bank and further prepared using Autodock-Tools. The grid box was centered on the STAT3 monomer, and the size of the grid box was adjusted to include the entire monomer. Docking experiments were performed at various default exhaustiveness values of 16, 24, 32, 40, and 60. Molecular graphics of the best binding model were generated using Discovery Studio Visualizer 2.0.

### 18) Statistical Analysis

Data were analyzed using GraphPad Prism software (Version 4.03; GraphPad software, Inc., San Diego, CA, USA). Data are presented as mean±SEM. All data differences were assessed by one-way analysis of variance (ANOVA). When the P value showed statistical significance in student's t-test, the difference was evaluated by Dunnett's test. Values of p < 0.05 were considered statistically significant.

### 2. Effect of bee venom extract in vitro

### 1) Determination of bvPLA2 effect on neuroinflammation and amyloidogenesis

Expression level changes in amyloid precursor protein (APP), beta-secretase 1 (BACE1), beta-amyloid (Aβ) in microglia (BV-2 cells) caused by bvPLA2 treatment were determined by Western blot. As a result, as shown in FIG. 6A, the levels of APP, BACE1, and β-Amyloid (Aβ) in cells increased due to LPS treatment, and the levels were decreased in a concentration-dependent manner upon treatment with bvPLA2.

Since β-amyloid was generated due to the activated β-secretase, β-secretase activity assay was performed. As a result, as shown in FIG. 6B, it was found that the β-secretase activity increased by LPS was decreased in a concentration-dependent manner upon treatment with bvPLA2.

The levels of inflammatory proteins such as iNOS and COX-2 and IBA-1, a marker of activated microglia, were determined by Western blot. As a result, as shown in FIG. 6C, the expression levels of iNOS, COX-2, and IBA-1 were increased upon treatment with LPS, and the expression levels of iNOS, COX-2, and IBA-1 were decreased in a concentration-dependent manner upon treatment with bvPLA2..

To determine whether or not bvPLA2 affects inhibition of NF-κB translocation, the level of the NF-κB subunit in the nucleus of BV-2 cells was measured by Western blot. As a result, as shown in FIG. 6D, the expression levels of both p65 and p50 were increased in the nucleus upon LPS treatment, but their expression levels were decreased in a concentration-dependent manner upon bvPLA2 treatment.

In addition, qRT-PCR was performed to determine whether or not bvPLA2 had an effect on reduction of pro-inflammatory cytokines that promote an inflammatory response. As a result, as shown in FIG. 6E, the levels of TNF-α, IL-1β, and IL-6, which are widely distributed in neurons among pro-inflammatory cytokines, were increased rapidly due to LPS treatment, and the levels of TNF-α, IL-1β, and IL-6 were all decreased in a concentration-dependent manner upon bvPLA2 treatment.

Based on this, it can be confirmed that bvPLA2 has an inhibitory effect on β-amyloid accumulation in microglia by lowering β-secretase activity and intracellular levels of APP and BACE1, and also has the effect of alleviating neuroinflammation by reducing intracellular levels of iNOS and COX-2 and reducing the expression level of pro-inflammatory cytokines.

### 2) Determination of combination effect of bee venom extract for neuroinflammation

To determine a combination effect of the bee venom extract and an NF-κB inhibitor on neuroinflammation, Western blot and qRT-PCR were performed by treating BV-2 cells with LPS (1 µg/mL) and bvPLA2 (100 ng/mL).

First, as a result of measuring the levels of iNOS and COX-2 related to neuroinflammation by Western blot, in the case that cells were treated with bvPLA2 and Bay 11-7802, which is an NF-κB inhibitor, individually, both iNOS and COX-2 were decreased, and in the case that cells were treated with bvPLA2 and the NF-κB inhibitor Bay 11-7802 together, the levels of iNOS and COX-2 were further decreased, as shown in FIG. 7A.

As a result of Western blot measurements of the intracellular levels of p-IκBα related to the NF-κB signaling pathway and the levels of p65 and p50 in the nucleus, the increases in p-IκBα, p65, and p50 caused by LPS treatment were all alleviated in the case that cells were treated with bvPLA2 and the NF-κB inhibitor (Bay 11-7802), individually, and the levels of p- IκBα, p65, and p50 were further decreased in the case that cells were treated with bvPLA2 and the NF-κB inhibitor (Bay 11-7802) together, as shown in FIG. 7B.

In order to investigate the effect of bvPLA2 and NF-κB inhibitors on the increase of TNF-α, IL-1β, and IL-6, which are proinflammatory cytokines caused by LPS treatment in BV-2 cells, an intracellular expression amount was measured by qRT-PCR. As a result, in the case that cells were treated with bvPLA2 and the NF-κB inhibitor (Bay 11-7802) individually, it can be determined that the expression levels of TNF-α, IL-1β, and IL-6 increased due to LPS were decreased, and in the case that cells were treated with these two together, it can be determined that the expression levels of pro-inflammatory cytokines were further decreased than those of the cells treated individually and to the level of the control group, as shown in FIG. 7C.

Therefore, it can be confirmed that the neuroinflammation alleviation effect of bvPLA2 in microglia cells (BV-2 cells) is greater when combined with the NF-κB inhibitor (Bay 11-7802).

### 3. Effect of bee venom extract in animal model of Alzheimer's disease - LPS-induced neuroinflammation in mice

### 1) Determination of memory relieving effect of bee venom extract

The Alzheimer's animal model in this example is a mouse in which neurogenic inflammation was induced by injection of LPS, and the memory loss and β-amyloid accumulation are similar to those of Alzheimer's patients.

In order to investigate whether or not bvPLA2 would be effective in treating Alzheimer's disease, Alzheimer's disease was induced by intraperitoneal injection of LPS at a dose of 250 µg/kg for one week, and bvPLA2 was administered by intraperitoneal injection 3 times a week (FIG. 8). Each injection was administered at regular time intervals of 2 hours or more without simultaneous administration, and behavioral experiments were conducted during induction and administration to measure the degree of memory decline, a typical symptom of Alzheimer's disease, along with administration of LPS and bvPLA2.

The Morris water maze experiment, a behavioral experiment often used to evaluate learning ability and spatial memory ability, was conducted twice a day for 6 days. As a result, as shown in FIGS. 9A and 9B, the control group moved 352.61 cm in an average of 24.64 seconds to find the platform on the 6th day, but the LPS group moved 502.79 cm in an average of 37 seconds to find the platform. On the other hand, it was found that the group administered with 2 mg/kg of bvPLA2 moved 229.97 cm in an average of 26.14 seconds to find the platform.

In other words, since the LPS group took a longer time to find the platform (longer escape latency) and moved a longer distance (longer escape distance) than the control group, it can be determined that the learning ability and the spatial memory ability were lowered. On the other hand, since the bvPLA2 administration group reduced the escape latency and the escape distance in a concentration-dependent manner, it can be determined that administration of bvPLA2 alleviated decline of the learning ability and spatial memory ability of the mouse due to the LPS treatment.

The next day after completion of the Morris water maze, a probe test was performed to evaluate the memory retention ability. As a result, the control group stayed on the target for an average of 24.83% for 60 seconds, but the LPS group stayed for an average of 17.79% on the target for the same time and the retention time was decreased, as shown in FIG. 9C. On the other hand, in the groups administered with bvPLA2 0.02, 0.2, and 2 mg/kg, the average retention time was 23.44%, 25.33%, and 27.29%, respectively, indicating that the retention time was increased compared to the LPS group.

As the last behavioral experiment, a passive avoidance test was performed to test how long the test subject remembered. As a result, there was no difference in the time taken to move from the illuminated compartment to the dark compartment for all groups in the training trial, but the control group stayed for an average of 69.63 seconds and the LPS group stayed for an average of 18.45 seconds when tested the next day, as shown in FIG. 9D. On the other hand, for the groups administered with 0.2 and 2 mg/kg of bvPLA2, the average retention time in the illuminated compartment was 46.92 and 67.59 seconds, respectively, showing that the retention time increased significantly compared to the LPS group.

Therefore, based on the behavioral experimental results in the Alzheimer's animal model, it can be determined that bvPLA2 alleviates declines in learning ability, spatial memory ability, memory retention ability, and memory, which are typical symptoms of Alzheimer's disease.

### 2) Determination of amyloidosis reduction effect of bee venom extract

In order to confirm whether or not the bee venom extract has an effect on amyloidosis, β-amyloid (Aβ) production in mouse brain tissues was measured by ELISA. As a result, β-amyloid was accumulated on average about 1.6 times more in the LPS group than in the control group, and the bvPLA2 administration group showed a concentration-dependent decrease in accumulation of β-amyloid, as shown in FIG. 10A.

Since β-amyloid (Aβ) is produced due to β-secretase that degrades amyloid precursor protein (APP), β-secretase activity assay to measure the activity of β-secretase was performed. As a result, the activity of β-secretase was increased in the LPS group than that of the control group, and the activity of β-secretase was decreased in a concentration-dependent manner due to bvPLA2 treatment, as shown in FIG. 10B.

In addition, as a result of determining changes in APP, BACE1, and β-amyloid (Aβ) levels caused by bvPLA2 treatment in mouse brain tissues by Western blot, APP, BACE1, and Aβ levels were rapidly increased in the LPS group, but the levels were decreased in a concentration-dependent manner in the bvPLA2 administration group, as shown in FIG. 10C.

Based on this, it can be confirmed that bvPLA2 reduces the activity of β-secretase that affects β-amyloid accumulation, thereby reducing the levels of APP, BACE1, and Aβ and alleviating Alzheimer's disease symptoms.

### 3) Determination of neuroinflammation effect of bee venom extract

To determine the effect of bee venom extract on changes in neuroinflammation in brain tissues of mice in which Alzheimer's disease was induced with LPS, the expression level of inflammatory proteins and the activity of astrocytes and microglial cells were investigated by the immunohistochemistry method.

As a result, as shown in FIG. 11A, it was found that the number of GFAP-reactive cells and IBA-1-reactive cells increased in the LPS group than in the control group, which showed that activation of astrocytes and microglial cells makes progress of neurodegenerative disorders. On the other hand, in the group administered with bvPLA2, the number of GFAP-responsive cells and IBA-1-reactive cells decreased in a concentration-dependent manner, showing that bvPLA2 inhibits the onset of neurodegenerative disorders such as Alzheimer's disease.

In addition, as shown in FIG. 11B, the number of iNOS-reactive cells and COX-2-reactive cells increased in the LPS group than in the control group, indicating that the brain tissues were inflamed due to LPS. On the other hand, iNOS-reactive cells and COX-2-reactive cells were decreased in a concentration-dependent manner in the brain tissues of mice treated with bvPLA2, indicating that bvPLA2 helps to alleviate neuroinflammatory responses.

### 4) Determination of effect of bee venom extract on changes in neuroinflammation

To investigate the effect of bee venom extract on changes in neuroinflammation in brain tissues of mice in which Alzheimer's disease was induced by LPS, the expression level of inflammatory proteins and the activity of astrocytes and microglia were determined by Western blot.

As a result, the expression levels of iNOS and COX-2 were significantly increased in the LPS group than in the control group, indicating that the brain tissue was inflamed due to LPS, as shown in FIG. 12A. On the other hand, it was found that the expression levels of iNOS and COX-2 decreased in a concentration-dependent manner in the brain tissues of mice treated with bvPLA2, indicating that bvPLA2 helps to alleviate neuroinflammatory responses.

In addition, the expression of GFAP (astrocyte activation marker) and IBA-1 (microglia activation marker) was increased in the LPS group than in the control group, indicating that neurodegenerative disease progresses due to the activation of astrocytes and microglia. On the other hand, in the group administered with bvPLA2, the expression of GFAP and IBA-1 was decreased, indicating that bvPLA2 inhibits the onset of neurodegenerative disorders such as Alzheimer's disease.

To determine whether or not bvPLA2 affects NF-κB translocation inhibition in mouse brain tissues, the levels of NF-κB subunits p50 and p65 in the nucleus of the hippocampus, and the level of p-IκBα in the cytosol were measured by Western blot.

As a result, both p65 and p50 levels in the nucleus increased in the LPS group, and the levels were decreased in a concentration-dependent manner in the case that bvPLA2 was administered, as shown in FIG. 12B. Also, in the case of p-IκBα, the level was increased in the LPS group, and the level was decreased in a concentration-dependent manner in the case that bvPLA2 was administered.

To investigate whether or not bvPLA2 affects decrease in the expression level of proinflammatory cytokines that promote inflammatory responses in mouse brain tissues, the expression levels of TNF-α, IL-1β, and IL-6 were measured and compared by ELISA.

As a result, the expression levels of TNF-α, IL-1β, and IL-6 were found to increase rapidly in the LPS group than in the control group, and in the case of being treated with bvPLA2, the levels of all TNF-α, IL-1β, and IL-6 significantly reduced in a concentration-dependent manner, as shown in FIG. 12C.

Taking these results together, it can be determined that administration of bvPLA2 helps to relieve neuroinflammation in the LPS-induced Alzheimer's mouse model.

### 5) Determination of effect of bee venom extract on Treg (regulatory T cell)

In order to examine effects of the bee venom extract on regulatory T cells that suppress the inflammatory response in brain tissues of mice in which Alzheimer's disease was induced by LPS, the level of Foxp3, a Treg marker, was determined by immunohistochemistry, qRT-PCR and Western blot.

As a result, the number of Foxp3-reactive cells was decreased in the LPS group than in the control group, indicating that inflammation due to LPS occurred, as shown in FIGS. 13A and 13B. In addition, it was found that the number of Foxp3-reactive cells increased in a concentration-dependent manner in the brain tissues of mice treated with bvPLA2, indicating that bvPLA2 helps to relieve neuroinflammation by activating Treg.

As a result of qRT-PCR to investigate whether or not bvPLA2 had an effect on Foxp3 expression in mouse brain tissues, the expression level of Foxp3 was decreased in the LPS group than in the control group, and it was found that the expression level of Foxp3 was recovered back to a level of the control group in a concentration-dependent manner upon bvPLA2 treatment, as shown in FIG. 13C.

As a result of Western blotting to determine whether or not there was a change in Foxp3 level in the mouse brain tissues, it was found that the expression level of Foxp3 increased in a concentration-dependent manner upon treatment with bvPLA2, as shown in FIG. 13D.

Based on this, it can be determined that bvPLA2 activates regulatory T cells to alleviate neuroinflammatory symptoms caused by Alzheimer's disease.

### 6) Comparison of effects of relief of memory loss and inhibition of β-amyloid (Aβ) accumulation according to administration route of bee venom extract

In order to compare the effects of relief of memory loss and inhibition of β-amyloid accumulation according to the route of administration of the bee venom extract, Alzheimer's disease was induced by intraperitoneal injection of LPS at a dose of 250 µg/kg daily for one week, and bvPLA2 was administered by intraperitoneal injection or subcutaneous injection at a dose of 0.2 mg/kg day 3 times a week (FIG. 8). Each injection was administered at regular time intervals of 2 hours or more without simultaneous administration, and behavioral experiments were conducted during induction and administration to measure the degree of memory decline, a typical symptom of Alzheimer's disease, along with administration of LPS and bvPLA2.

As a result of conducting the Morris water maze, a behavioral experiment frequently used to evaluate learning ability and spatial memory ability, twice a day for 6 days, on the 6th day, the control group found the platform by moving 352.61 cm in an average of 24.64 seconds, as shown in FIGS. 14A and 14B. But the LPS group found the platform by moving 502.79 cm in an average of 37 seconds. On the other hand, in the group administered with bvPLA2 0.2 mg/kg intraperitoneally, it moved 404.9 cm in an average of 28.94 seconds, and the group administered by subcutaneous injection moved 319.6 cm in an average of 22.08 seconds to find the platform.

Therefore, it can be determined that when bvPLA2 was administered by subcutaneous injection (0.2 mg/kg), the effect of alleviating decrease in learning ability and spatial memory ability of mice was greater than when administered by intraperitoneal injection.

As a result of conducting the probe test to evaluate the memory retention ability on the next day after the Morris water maze was completed, the control group stayed on the target for an average of 24.83% for 60 seconds, but the LPS group decreased the retention time by staying on the target for an average of 17.79% for the same period of time, as shown in FIG. 14C. On the other hand, in the group administered by intraperitoneal injection of bvPLA2 0.2 mg/kg, the average retention time was 25.33%, and in the group administered by subcutaneous injection, the average retention time was 26.22%. Thus, it was determined that the retention time was increased in the group administered by subcutaneous injection compared to the group administered by intraperitoneal injection.

As a result of the passive avoidance experiment to test how long the test subject remembered as the last behavioral experiment, in the training trial of all groups, there was no difference in the time taken for the mice to move from the illuminated compartment to the dark compartment, as shown in FIG. 14D. However, when tested the next day, the control group stayed for an average of 69.63 seconds, and the LPS group stayed for an average of 18.45 seconds. On the other hand, the group administered by intraperitoneal injection of bvPLA2 0.2 mg/kg stayed for an average of 46.92 seconds, and the group administered subcutaneously stayed for an average of 38.03 seconds. Thus, it was determined that the retention time in the illuminated compartment was longer in the group administered by intraperitoneal injection.

Therefore, considering the above behavioral experimental results in the Alzheimer's disease animal model, it was determined that bvPLA2 could have a greater effect in relieving the typical symptoms of Alzheimer's disease, such as the decline of learning ability, spatial memory ability, memory retention ability, and memory capacity when administered by subcutaneous injection, compared to intraperitoneal injection administration at the same dose.

In addition, in order to determine whether or not the difference in the administration method of bvPLA2 exhibited a difference in the effect on amyloidosis, β-amyloid production in mouse brain tissues was measured by ELISA. As a result, it was determined that the level of β-amyloid was significantly increased in the LPS group than in the control group, and decreased when treated with bvPLA2, as shown in FIG. 14E.

In particular, the β-amyloid level of the subcutaneous injection group was lower than that of the intraperitoneal injection group, and it was determined that when bvPLA2 was administered by subcutaneous injection, the level was reduced to a level similar to that of the control group.

### 4. Effect of bee venom extract in animal model of Alzheimer's disease - Tg2576 mice

### 1) Selection and treatment of experimental animals

The Alzheimer's disease animal model used in this example is a dementia-inducing transgenic Tg2576 mouse (swAPP; a mouse having a double mutation (K670N/M671L) found in a Swedish family with early-onset familial Alzheimer's Disease), which is widely used as an Alzheimer's disease mouse model, and shows an age-dependent increase in β -amyloid (Aβ) and the production of amyloid plaques. The accumulation of amyloid in the brain of Tg2576 mice is characterized by a decrease in the expression of synaptophysin protein and a decrease in the gene expression of proteins involved in neural synapse formation, cytoskeleton formation, and metabolism.

In an Aβ infusion model, an Aβ infusion pump was injected into one hemisphere of the mouse brain so that the concentration of Aβ in the brain was maintained at 300 pmol for 14 days to induce neurodegeneration and impairment of brain function accompanied by impairment of learning and memory. This can be replaced by Tg2576 because Tg2576 expresses the human APP 695 (swAPP) gene and causes amyloid accumulation in the brain. Whereas the Aβ infusion model simply causes damage to brain function due to Aβ-induced neurotoxicity, the Tg2576 mouse causes damage to brain function due to a complex mechanism by the gene and thus is more suitable for the development of Alzheimer's disease treatment.

In addition, the swAPP/PS2 mouse contains the same swAPP gene as the Tg2576 mouse model, which is a model expressing the mutant PS2 gene and causes amyloid accumulation genetically, and is used as a mouse model of Alzheimer's disease. Since it is possible to replace this with Tg2576 in that it exhibits symptoms of Alzheimer's dementia due to congenital amyloid accumulation, in this example, Tg2576 mice were selected as the Alzheimer's disease animal model.

12-month-old Tg2576 mice were managed and handled according to the Humane Animal Care and Use Guidelines of the Ministry of Food and Drug Safety. Tg2576 mice having human APP695 carrying the Swedish double mutation (hAPP; HuAPP695; K670 N/M671 L) were purchased from Taconic Farms (Germantown, NY, USA), and the strain was maintained in the animal laboratory of Chungbuk National University of Korea. Tg2576 mice were randomly divided into two groups, a control group and a group treated with bvPLA2 (1 mg/kg), with 10 mice in each group.

bvPLA2 was administered intraperitoneally twice per week for 4 weeks. Alternatively, control mice were administered with vehicle at the same dose. Behavioral experiments of learning and memory abilities were assessed using a water maze, probe and passive avoidance experiments. Mice were sacrificed after the behavioral experiments by CO₂ asphyxiation.

### 2) Determination of alleviation effect of bee venom extract on genetically induced memory impairment in Tg2576 mice

The Tg2576 mouse, which is the AD (Alzheimer's Disease) mouse model used in this example, overexpresses human APP with the mutation found in familial AD in Sweden. Tg2576 mice rapidly produce Aβ by up to 6 months of age and then begin to produce Aβ plaques between 9 and 12 months of age. At 13 months, the level of Aβ plaques builds up rapidly and the mice show symptoms of AD, such as impaired cognitive ability.

In order to determine a memory improvement effect of the bee venom extract in the Tg2576 AD mouse model, bvPLA2 (1 mg/kg) was administered to mice by intraperitoneal injection twice a week for 4 weeks (FIG. 15A). Four weeks after administration, the Morris water maze, probe, and passive avoidance experiments were successively performed to evaluate learning ability and spatial memory ability. Escape latency and distance were measured to determine the effect of bvPLA2 on memory improvement.

As a result, the average escape latency and swimming distance of the control group on the 6th day were about 29.71±4.189 seconds and 346.1±71.43 cm, respectively, as shown in FIGS. 15B and 15C. On the other hand, the average escape latency and distance of the bvPLA2 administration group were 19.36±2.790 seconds and 186.8±28.48 cm, respectively, which were significantly reduced compared to the control group.

The next day after completion of the Morris water maze, the probe test was performed to evaluate the memory retention ability. As a result, as shown in FIG. 15D, the average retention time in the target quadrant was significantly increased in the bvPLA2 administration group (30.54±4.992%) compared to the control group (12.31±3.355%).

As a result of the passive avoidance experiment 2 days after the probe test, as shown in FIG. 15E, the control group showed an average step-through retention time of 36.17±8.310 seconds in the illuminated compartment, and the bvPLA2 administration group showed the time of 107.5±20.90 seconds, which was significantly increased compared to the control group.

### 3) Determination of Aβ accumulation inhibitory effect of bee venom extract

The best known cause of AD is Aβ accumulation. Therefore, Thioflavin S staining was performed to determine effects of the bee venom extract on the genetically induced Aβ accumulation in the brain of Tg2576 mice. Thioflavin S staining is mainly used to stain β-sheet-rich structures of Aβ found in Aβ plaques. As a result, as shown in FIG. 16A, it was found that the accumulation of Aβ plaques was significantly reduced in the bvPLA2 administration group compared to the control group.

The levels of APP and BACE1 involved in Aβ production were detected using Western blot analysis of Tg2576 mouse brain. As a result, as shown in FIG. 16B, it was found that the expression levels of APP and BACE1 decreased in the bvPLA2 administration group compared to the control group.

ELISA was performed to quantitatively measure Ap₁₋₄₂ levels and Aβ₁₋₄₀ levels in Tg2576 mouse brain. As a result, as shown in FIG. 16C, the Ap₁₋₄₂ level of the control group was 3039±116.8 pg/mg of protein in the brain, and the Ap₁₋₄₂ level of the bvPLA2 administration group was 2236±244.9 pg/mg of protein. In addition, as shown in FIG. 16D, the Aβ₁₋₄₀ level of the control group was 2182±168.6 pg/mg in the brain, and the Aβ₁₋₄₀ level of the bvPLA2 administration group was 1593±53.25 pg/mg. Comparing the amyloid levels between the two groups, it was found that bvPLA2 administration significantly reduced the Aβ level in the brain of Tg2576 mice.

To determine how amyloid production is reduced by bvPLA2, β-secretase activity in the brain was analyzed. As a result, as shown in FIG. 16E, it was determined that the β-secretase activity was significantly reduced by administration of bvPLA2.

The effect of bvPLA2 on cell viability in BV-2 cells was determined by MTT assay (FIG. 18A). Since the group treated with bvPLA2 (0.01, 0.1, 1 µg/mL) did not show a significant change in cell viability compared to the control group, it was determined that bvPLA2 was not toxic up to 1 µg/mL in BV-2 cells.

To investigate the effect of bvPLA2 on amyloidogenesis in vitro, BV-2 cells were treated with LPS and various concentrations of bvPLA2 (0.01, 0.1, 1 µg/mL) for 24 hours. Aβ₁₋₄₂ levels in BV-2 cells were determined by ELISA.

As a result, as shown in FIG. 16F, the Aβ₁₋₄₂ level in the control group was 144.9±3.587 pg/mg, and in the LPS group, it was significantly increased to 356.5±6.030 pg/mg. However, the bvPLA2-treated group showed a concentration-dependent decrease to 273.7±23.33 pg/mg for the bvPLA2 1 µg/mL treatment group.

Referring to FIG. 16G, β-secretase activity was also significantly increased in the LPS group compared to the control group, but it was found that the activity was decreased in a concentration-dependent manner by treatment with bvPLA2.

Expression levels of APP and BACE1 were measured by Western blot analysis. As a result, as shown in FIG. 16H, the expression of APP and BACE1 was significantly increased in the LPS group compared to the control group, whereas it was decreased in the bvPLA2-treated group.

### 4) Determination of effect of bee venom extract on neuroinflammation in Tg2576 mouse brain

Another hallmark of AD is activation of astrocytes and microglia (i.e. neuroinflammation). Immunohistochemistry and Western blot were performed to investigate the expression level of neuroinflammation-related proteins in the mouse brain.

As a result, as shown in FIGS. 17A and 17B, the numbers of GFAP and IBA-1, which are markers of reactive astrocytes and activated microglia, respectively, were decreased in the mice administered with bvPLA2 compared to the control group, and the numbers of iNOS and COX-2, the inflammatory proteins, were also decreased in the bvPLA2 group.

In addition, as shown in FIG. 17C , the expression levels of iNOS and COX-2 were decreased in the brains of Tg2576 mice administered with bvPLA2, and the expression levels of GFAP and IBA-1 were also significantly decreased in the brains of the bvPLA2 administration group.

The production of pro-inflammatory cytokines is well known as a marker of development of inflammation, and the production of anti-inflammatory cytokines indicates decrease in inflammation. Therefore, qRT-PCR was performed to determine the production levels of pro-inflammatory and anti-inflammatory cytokines.

As a result, as shown in FIG. 17D, mRNA levels of proinflammatory cytokines such as TNF-α, IL-1β, and IL-6 were decreased in the brain of the bvPLA2 administration group compared to the control group. On the other hand, mRNA levels of anti-inflammatory cytokines such as IL-4 and TGF-β were significantly increased in the bvPLA2 administration group compared to the control group. However, the expression level of the anti-inflammatory cytokine, IL-10, was decreased in the brain of the mice of the bvPLA2 administration group.

### 5) Determination of effect of bee venom extract on neuroinflammation in BV-2 microglia

An anti-inflammatory effect of bvPLA2 in microglia was investigated through nitric oxide concentration and expression levels of inflammation-related proteins, and pro-inflammatory and anti-inflammatory cytokines.

Nitric oxide (NO) is secreted from activated macrophages, and nitric oxide assay was performed to determine the degree of relief of neuroinflammation in BV-2 cells due to treatment with bvPLA2. As a result, as shown in FIG. 18B, the nitric oxide concentration was significantly reduced to 14.92±2.154% in the bvPLA2-treated group compared to the LPS group.

Referring to FIG. 18C, the expression of iNOS, COX-2, and IBA-1 was significantly decreased in a concentration-dependent manner in the bvPLA2-treated group compared to the LPS group. Referring to FIG. 18D, the expression levels of proinflammatory cytokines such as TNF-α, IL-1β, and IL-6 were significantly increased in the LPS group and decreased in a concentration-dependent manner in the bvPLA2-treated group, whereas the expression levels of anti-inflammatory cytokines such as IL-4 and TGF-β were significantly decreased in the LPS group, but were recovered in a concentration-dependent manner in the bvPLA2-treated group.

Although not shown in the drawings, Western blot analysis and qRT-PCR were performed to determine whether or not bvPLA2 had an inhibitory effect on Aβ-induced neuroinflammation. As a result, the expression levels of iNOS, COX-2, and p-STAT3 were significantly decreased in a concentration-dependent manner in the bvPLA2-treated group compared to the Aβ treated group. The levels of proinflammatory cytokines (TNF-a, IL-1β, and IL-6) were significantly increased by Aβ treatment and decreased in a concentration-dependent manner in the bvPLA2-treated group. Based on this, it can be determined that bvPLA2 has an inhibitory effect on the inflammatory response induced by LPS or Aβ in BV-2 cells.

### 6) Determination of inhibitory effect of bee venom extract on STAT3 activation through binding of bvPLA2 and STAT3

To investigate which signaling pathways are involved in the anti-neuroinflammatory effect of bvPLA2, the levels of factors related to STAT3 and mitogen-activated protein kinases (MAPK) signaling pathways, which are highly related to inflammation, were determined using Western blotting in the brain of Tg2576 mice.

As a result, as shown in FIG. 19A, the level of p-STAT3 was significantly decreased in the bvPLA2 administration group, and only the level of p-ERK in the MAPK signal was significantly decreased.

From this, it can be seen that bvPLA2 was closely related to the STAT3 signaling pathway. Accordingly, the level of p-STAT3 was evaluated by treating the BV-2 cells activated by LPS (1 µg/mL) with bvPLA2 (0.01, 0.1, 1 µg/mL).

Referring to FIG. 19B, the Western blot analysis showed that the level of p-STAT3 was significantly increased by the LPS treatment as compared to the control group, but it was decreased by the bvPLA2 treatment in a concentration-dependent manner. In particular, when treated with bvPLA2 1 µg/mL, the level of p-STAT3 was reduced to a level similar to that of the control group.

To determine an effect of bvPLA2 on translational activity for the STAT3 promoter, luciferase activity assay was performed. As a result, as shown in FIG. 19C, it was determined that bvPLA2 reduced the STAT3 luciferase activity in a concentration-dependent manner in BV-2 cells.

To determine whether or not the effect of bvPLA2 was related to binding to STAT3, a pull-down assay and docking experiment between bvPLA2 and STAT3 were performed. Whole cell lysates from BV-2 cells overexpressing STAT3 were incubated with Sepharose 4B beads and bvPLA2-conjugated Sepharose 4B beads, and then detection was performed by immunoblotting with an anti-STAT3 antibody.

As a result, as shown in FIG. 19D, the STAT3 protein level was higher in bvPLA2-conjugated Sepharose 4B beads, indicating that bvPLA2 could directly bind to STAT3.

### 7) Bee venom extract directly binding to linker domain (LD) of STAT3

To identify the binding site where bvPLA2 binds to STAT3, computational molecular docking modeling between bvPLA2 and STAT3 was performed. As a result, as shown in FIG. 20A, it was found that bvPLA2 bound directly to several amino acids of STAT3, in particular, a linker domain (LD) of STAT3 (the inside of the binding pocket consisting of Thr500, Asp502, Gln503, Glu506, Ser509, Trp510, Ser513, Lys517, Arg518, Leu520, and Ile522).

To determine which domain of STAT3 interacts with bvPLA2, pull-down analysis, luciferase activity analysis, and qRT-PCR were performed using STAT3 in several mutant forms such as wild type, DNA binding domain (DBD)-null, and LD-null (FIG. 20B).

To determine the interaction between bvPLA2 and LD of STAT3, pull-down analysis was performed using bvPLA2-conjugated Sepharose 4B beads and cell lysates transfected with wild type (WT)-STAT3, DBD-null STAT3, and LD-null STAT3. As a result, as shown in FIG. 20C, bvPLA2-conjugated Sepharose 4B beads pulled down WT-STAT3 and DBD-null STAT3, but did not pull down LD-null STAT3.

Referring to FIG. 20D, the luciferase activity of STAT3 was significantly increased by LPS treatment in all of the BV-2 cells transfected with WT-STAT3, DBD-null STAT3, or LD-null STAT3, and bvPLA2 reduced STAT3 luciferase activity in BV-2 cells of WT-STAT3 vector or DBD-null STAT3 vector except for LD-null STAT3 vector.

To determine whether the anti-inflammatory effect of bvPLA2 was different in BV-2 cells transfected with WT-STAT3, DBD-null STAT3, or LD-null STAT3, qRT-PCR was performed. As a result, as shown in FIG. 20E, in BV-2 cells transfected with WT-STAT3 or DBD-null STAT3, the levels of TNF-α, IL-1β, and IL-6 were decreased by bvPLA2. However, in BV-2 cells transfected with LD-null STAT3, the level of the cytokines was hardly affected by bvPLA2 treatment. Based on this, it was determined that bvPLA2 could inhibit the activation of STAT3 by binding to the LD of STAT3.

### 8) Determination of synergistic effect of bee venom extract and STAT3 inhibitor on amyloid production and neuroinflammation

The inhibitory effects of a STAT3 inhibitor (Stattic) and the bee venom extracts on amyloid production and neuroinflammation were tested in BV-2 cells. To determine the combination effect of bvPLA2 and Stattic on amyloidogenesis and neuroinflammation in vitro, BV-2 microglia were treated with LPS (1 µg/mL), Stattic (1 µM), or bvPLA2 (1 µg/mL) for 24 h.

Ap₁₋₄₂ levels of BV-2 cells were measured by ELISA. Referring to FIG. 21A, the Aβ level was found to be 264.2±4.384 pg/mg in the Stattic-treated group, and 273.7±23.33 pg/mg in the bvPLA2-treated group. These levels were significantly reduced to 199.4±20.39 pg/mg of protein in the group co-treated with both Stattic and bvPLA2.

Referring to FIG. 21B, β-secretase activity, which acts directly on Aβ production, was significantly increased by LPS treatment compared to the control group, decreased by treatment with Stattic or bvPLA2, and significantly decreased by co-treatment of Stattic and bvPLA2.

Furthermore, as shown in FIG. 21C , it was determined by Western blot that the increased expression levels of Aβ and BACE1 by LPS treatment in BV-2 cells were decreased by bvPLA2 treatment, but more significantly decreased when the co-treatment was performed.

To investigate effects of combination of Stattic and bvPLA2 on neuroinflammation, the levels of inflammatory proteins and pro-inflammatory cytokines in BV-2 cells were evaluated. As a result, as shown in FIG. 21D, the intracellular levels of inflammatory proteins such as iNOS and COX-2 increased by LPS treatment were decreased by bvPLA2 or Stattic, and when bvPLA2 or Stattic were used together, they were further decreased.

In addition, as shown in FIG. 21E, the expression levels of TNF-α, IL-1β, and IL-6 increased by LPS treatment were decreased by treatment with bvPLA2 or Stattic, and when treated with them together, the levels of TNF-α, IL-1β, and IL-6 were decreased more significantly than those when treated with Stattic or bvPLA2 alone.

Although the embodiments and examples of the present invention have been described in detail above, for those of ordinary skill in the art, it is clear that these specific descriptions are merely preferred embodiments and the scope of the present invention is not limited thereby. In other words, the substantial scope of the present invention is defined by the appended claims and their equivalents.

## Claims

1. A pharmaceutical composition for preventing or treating neuroinflammatory disorders comprising a bee venom extract having a phospholipase A2 (PLA2) content of 50 to 85% as an active ingredient.

2. The pharmaceutical composition for preventing or treating neuroinflammatory disorders according to claim 1, wherein the bee venom extract improves motor function deterioration due to neurotoxicity.

3. The pharmaceutical composition for preventing or treating neuroinflammatory disorders according to claim 1, wherein the bee venom extract has a neuroprotective effect by reducing the polarization of Th1 and Th17 and increasing TH (tyrosine hydroxylase)-positive dopaminergic neurons.

4. The pharmaceutical composition for preventing or treating neuroinflammatory disorders according to claim 1, wherein the bee venom extract reduces accumulation of beta-amyloid (β-amyloid).

5. The pharmaceutical composition for preventing or treating neuroinflammatory disorders according to claim 1, wherein the composition is administered by a subcutaneous route.

6. The pharmaceutical composition for preventing or treating neuroinflammatory disorders according to claim 5, wherein the bee venom extract is administered in an amount of 3.75 mg/kg or more and less than 7.5 mg/kg.

7. The pharmaceutical composition for preventing or treating neuroinflammatory disorders according to claim 1, wherein the neuroinflammatory disorder is selected from the group consisting of Alzheimer's disease, vascular dementia, frontotemporal dementia, alcoholic dementia, Parkinson's disease, and stroke.

8. A pharmaceutical composition for subcutaneous administration for preventing or treating neuroinflammatory disorders, comprising a bee venom extract having a phospholipase A2 (PLA2) content of 50 to 85% as an active ingredient.

9. The pharmaceutical composition for subcutaneous administration for preventing or treating neuroinflammatory disorders according to claim 8, wherein the bee venom extract is administered subcutaneously in an amount of 3.75 mg/kg or more and less than 7.5 mg/kg.

10. A pharmaceutical composition for preventing or treating neuroinflammatory disorders comprising the composition according to claim 1 and one or more inhibitors selected from STAT3 inhibitors and NF-κB inhibitors.

11. A method of subcutaneously administering the composition according to any one of claims 1 to 10 for preventing or treating neuroinflammatory disorders.

12. A functional food composition for preventing or improving neuroinflammatory disorders, comprising a bee venom extract having a phospholipase A2 (PLA2) content of 50 to 85% as an active ingredient.

13. A functional food composition for preventing or improving neuroinflammatory disorders, comprising the composition according to claim 12 and one or more inhibitors selected from STAT3 inhibitors and NF-κB inhibitors.
